(19) Europäisches Patentamt
European Patent Office
Office européen des brevets

(11) EP 0 848 710 B1

(12) **EUROPÄISCHE PATENTSCHRIFT**

(45) Veröffentlichungstag und Bekanntmachung des Hinweises auf die Patenterteilung:
**10.08.2005 Patentblatt 2005/32**

(51) Int Cl.[7]: **C07D 251/18**, A01N 43/70, C07D 251/16, C07D 251/22, C07D 405/06

(21) Anmeldenummer: **96927678.1**

(22) Anmeldetag: **05.08.1996**

(86) Internationale Anmeldenummer:
**PCT/EP1996/003442**

(87) Internationale Veröffentlichungsnummer:
**WO 1997/008156 (06.03.1997 Gazette 1997/11)**

(54) **2,4-DIAMINO-1,3,5-TRIAZINE, VERFAHREN ZU DEREN HERSTELLUNG UND DEREN VERWENDUNG ALS HERBIZIDE UND PFLANZENWACHSTUMSREGULATOREN**

2,4- DIAMINO-1,3,5-TRIAZINES, PROCESS FOR THE PRODUCTION THEREOF, AND THE USE THEREOF AS HERBICIDES AND PLANT GROWTH REGULATORS

2,4-DIAMINO-1,3,5-TRIAZINES, LEUR PROCEDE DE PRODUCTION ET LEUR UTILISATION COMME HERBICIDES ET REGULATEURS DE LA CROISSANCE DE VEGETAUX

(84) Benannte Vertragsstaaten:
**AT BE DE ES FR GB GR IT NL PT SE**

(30) Priorität: **24.08.1995 DE 19531084**

(43) Veröffentlichungstag der Anmeldung:
**24.06.1998 Patentblatt 1998/26**

(73) Patentinhaber: **Bayer CropScience GmbH
65929 Frankfurt/Main (DE)**

(72) Erfinder:
- **GIENCKE, Wolfgang
D-65719 Hofheim (DE)**
- **MINN, Klemens
D-65795 Hattersheim (DE)**
- **WILLMS, Lothar
D-65719 Hofheim (DE)**
- **BIERINGER, Hermann
D-65817 Eppstein (DE)**
- **BAUER, Klaus
D-63456 Hanau (DE)**
- **ROSINGER, Christopher
D-65719 Hofheim (DE)**

(56) Entgegenhaltungen:
**EP-A- 0 356 412**
**EP-A- 0 411 153**
**WO-A-95/06642**
**US-A- 3 758 471**
**US-A- 3 816 419**

- **DATABASE WPI Section Ch, Week 7746 Derwent Publications Ltd., London, GB; Class B03, AN 77-81704Y XP002016136 & JP 52 083 582 A (MITSUBISHI CHEM IND KK) , 12.Juli 1977**
- **CHEMICAL ABSTRACTS, vol. 110, no. 13, 27.März 1989 Columbus, Ohio, US; abstract no. 114801, KELAREV V I ET AL: "Synthesis and properties of sym-triazine derivatives. 8. Synthesis of amino- and alkoxy-substituted sym-triazines containing fragments of sterically hindered phenol starting from 2,4-bis(trichloromethyl)-sym -triazines" XP002016129 & KHIM. GETEROTSIKL. SOEDIN. (KGSSAQ,04538234);88; (5); PP.681-6, MOSK. INST. NEFTI GAZA;MOSCOW; USSR (SU),**
- **CHEMICAL ABSTRACTS, vol. 086, no. 7, 14.Februar 1977 Columbus, Ohio, US; abstract no. 037551, TOBE A: "Pharmacological studies of triazine derivatives. I. General pharmacological actions" XP002016130 & YAKUGAKU ZASSHI (YKKZAJ);76; VOL.96 (2); PP.223-31, MITSUBISHI CHEM. IND., LTD.;CENT. RES. LAB.; KAWASAKI; JAPAN,**
- **CHEMICAL ABSTRACTS, vol. 090, no. 3, 15.Januar 1979 Columbus, Ohio, US; abstract no. 022979, SLUKA J ET AL: "2,4-Diamino-6-phenyl-1,3,5-triazines" XP002016131 & COLLECT. CZECH. CHEM. COMMUN. (CCCCAK,0366547X);78; VOL.43 (6); PP.1639-46, RES. INST. PHARM. BIOCHEM.;PRAGUE; CZECH.,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- **CHEMICAL ABSTRACTS, vol. 089, no. 5, 31.Juli 1978 Columbus, Ohio, US; abstract no. 043338, SCIORTINO T ET AL: "New substituted s-triazines of possible pharmacological interest" XP002016132 & BOLL. CHIM. FARM. (BCFAAI,00066648);77; VOL.116 (11); PP.637-43, UNIV. TRIESTE;IST. TEC. FARM.; TRIESTE; ITALY,**
- **CHEMICAL ABSTRACTS, vol. 088, no. 17, 24.April 1978 Columbus, Ohio, US; abstract no. 121115, OVSEPYAN T R ET AL: "Synthesis and mutagenic action of 6-chloromethyl, cyanomethyl, and.beta.-aminoethyl derivatives of substituted 1,3,5-triazines" XP002016133 & KHIM.-FARM. ZH. (KHFZAN,00231134);77; VOL.11 (12); PP.35-9, INST. TONKOI ORG. KHIM. IM. MNDZHOYANA;YEREVAN; USSR,**
- **CHEMICAL ABSTRACTS, vol. 079, no. 17, 29.Oktober 1973 Columbus, Ohio, US; abstract no. 105207, GUIOCA M V: "New substituted triazines and their diuretic activity" XP002016134 & ANN. PHARM. FR. (APFRAD);73; VOL.31 (4); PP.283-92, UNIV. ATHENS;LAB. PHARM. CHEM.; ATHENS; GREECE,**
- **CHEMICAL ABSTRACTS, vol. 079, no. 15, 15.Oktober 1973 Columbus, Ohio, US; abstract no. 092165, COLAUTTI A ET AL: "Substituted S-triazines and amidinoureas of potential pharmacological activity" XP002016135 & FARMACO, ED. SCI. (FRPSAX);73; VOL.28 (7); PP.531-8, UNIV. TRIESTE;IST. CHIM. FARM. TOSSICOL.; TRIESTE; ITALY,**


Bemerkungen:

Die Akte enthält technische Angaben, die nach dem Eingang der Anmeldung eingereicht wurden und die nicht in dieser Patentschrift enthalten sind.

## Beschreibung

Die Erfindung betrifft das technische Gebiet der Herbizide und Pflanzenwachstumsregulatoren, insbesondere der Herbizide zur selektiven Bekämpfung von Unkräutern und Ungräsern in Nutzpflanzenkulturen.

Es ist bekannt, daß in 6-Stellung substituierte 2-Amino-4-(phenoxyalkyl-amino)-1,3,5-triazine herbizide und pflanzenwachstumsregulierende Eigenschaften besitzen; vgl. WO 94/24086, EP-A-509544, EP-A-492,615. Weiterhin sind bereits 2-Amino-4-[arylamino- oder (hetero)arylalkyl-amino]-6-haloalkyl-1,3,5-triazine mit herbizider Wirkung bekannt; vgl. US-A-3,816,419, WO 90/09378, WO 88/02368.

Die bekannten Wirkstoffe mit einer 4-[(Hetero)arylalkyl-amino]-gruppe enthalten dabei als "Alkyl" jeweils eine Methylenbrücke, welche gegebenenfalls noch verzweigt substituiert ist.

Die bekannten Wirkstoffe weisen bei ihrer Anwendung teilweise Nachteile auf, sei es unzureichende herbizide Wirkung gegen Schadpflanzen, zu geringes Spektrum der Schadpflanzen, das mit einem Wirkstoff bekämpft werden kann, oder zu geringe Selektivität in Nutzpflanzenkulturen. Andere Wirkstoffe lassen sich wegen schwer zugänglicher Vorprodukte und Reagenzien im industriellen Maßstab nicht wirtschaftlich herstellen oder besitzen nur unzureichende chemische Stabilitäten.

Aufgabe der Erfindung ist es, alternative Wirkstoffe vom Typ der 2,4-Diamino-1,3,5-Triazine bereitzustellen, die als Herbizide oder Pflanzenwachstumsregulatoren eingesetzt werden können.

In der Deutschen Patentanmeldung Nr. 19 522 137.0 werden unter anderen Herbizide des genannten Typs vorgeschlagen, die in 6-Stellung am Triazinring einen gegebenenfalls substituierten Cycloalkylrest oder einen Heterocyclus mit einem Sauerstoff-, Stickstoff- oder Schwefelatom enthalten und in 4-Stellung einen Phenylalkylrest mit einer linearen Propylenbrücke aufweisen, die gegebenenfalls noch verzweigt substituiert ist.

Gegenstand der vorliegenden Erfindung sind Verbindungen der Formel (I) und deren Salze,

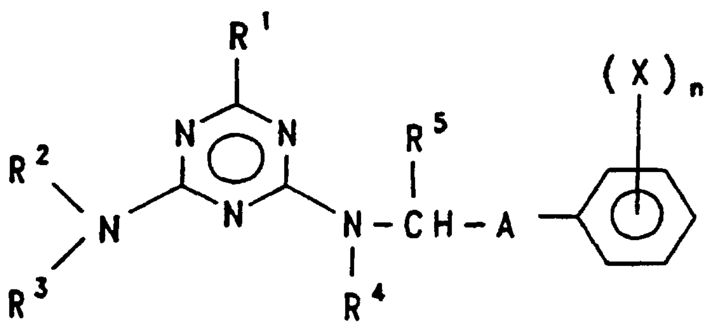

(I)

worin

$R^1$ $(C_1-C_4)$Alkyl,

das unsubstituiert oder durch einen oder mehrere Reste aus der Gruppe Halogen, $(C_1-C_4)$Alkoxy, $(C_1-C_4)$Alkylthio, $(C_1-C_4)$Alkylsulfonyl und $(C_3-C_9)$Cycloalkyl, das unsubstituiert oder substituiert ist, und Phenyl, das unsubstituiert oder durch einen oder mehrere Reste aus der Gruppe Halogen, $(C_1-C_4)$Alkyl und $(C_1-C_4)$Haloalkyl, $(C_1-C_4)$Alkoxy, $(C_1-C_4)$Haloalkoxy, $(C_1-C_4)$Alkylthio, Amino, Mono- und Di[$(C_1-C_4)$alkyl]amino, $(C_1-C_4)$Alkanoylamino, Benzoylamino, Nitro, Cyano, [$(C_1-C_4)$Alkyl]carbonyl, Formyl, Carbamoyl, Mono- und Di-[$(C_1-C_4)$alkyl]aminocarbonyl und $(C_1-C_4)$Alkylsulfonyl substituiert ist, und Heterocyclyl mit 3 bis 6 Ringatomen und 1 bis 3 Heteroringatomen aus der Gruppe N, O und S, wobei der Ring unsubstituiert oder durch einen oder mehrere Reste aus der Gruppe Halogen, $(C_1-C_4)$Alkyl und Oxo substituiert ist,

substituiert ist, oder

Phenyl, das unsubstituiert oder durch einen oder mehrere Reste aus der Gruppe Halogen, $(C_1-C_4)$Alkyl, $(C_1-C_4)$Haloalkyl, $(C_1-C_4)$Alkoxy, $(C_1-C_4)$Haloalkoxy und Nitro substituiert ist,

$R^2$ und $R^3$ jeweils unabhängig voneinander Wasserstoff, Amino oder Alkylamino oder Dialkylamino mit jeweils 1 bis 4 C-Atomen im Alkylrest, einen acyclischen oder cyclischen Kohlenwasserstoffrest oder Kohlenwasserstoffoxyrest mit jeweils 1 bis 6 C-Atomen oder

einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder durch einen oder mehrere Reste aus der Gruppe Halogen, $(C_1$-$C_4)$Alkoxy, $(C_1$-$C_4)$Haloalkoxy, $(C_1$-$C_4)$Alkylthio, $(C_2$-$C_4)$Alkenyl, $(C_2$-$C_4)$Alkinyl, $(C_2$-$C_4)$Alkenyloxy, $(C_2$-$C_4)$Alkinyloxy, Hydroxy, Amino, Acylamino, Mono- und Dialkylamino, Nitro, Carboxy, Cyano, Azido, $[(C_1$-$C_4)$Alkoxy]-carbonyl, $[(C_1$-$C_4)$Alkyl]-carbonyl, Formyl, Carbamoyl, Mono- und Di-$[(C_1$-$C_4)$alkyl]-aminocarbonyl, $(C_1$-$C_4)$Alkylsulfinyl, $(C_1$-$C_4)$Haloalkylsulfinyl, $(C_1$-$C_4)$Alkylsulfonyl, $(C_1$-$C_4)$Haloalkylsulfonyl und, im Falle cyclischer Reste, auch $(C_1$-$C_4)$Alkyl und $(C_1$-$C_4)$Haloalkyl substituiert ist,
oder einen Acylrest oder
$R^2$ und $R^3$ gemeinsam mit dem Stickstoffatom der Gruppe $NR^2R^3$ einen heterocyclischen Rest mit 3 bis 6 Ringatomen und 1 bis 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, $(C_1$-$C_4)$Alkyl und Oxo substituiert ist,

$R^4$ Wasserstoff, Amino, Alkylamino oder Dialkylamino mit jeweils 1 bis 6 C-Atomen im Alkylrest, einen acyclischen oder cyclischen. Kohlenwasserstoffrest oder Kohlenwasserstoffoxyrest mit jeweils 1 bis 6 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder durch einen oder mehrere Reste aus der Gruppe Halogen, $(C_1$-$C_4)$Alkoxy, $(C_1$-$C_4)$Haloalkoxy, $(C_1$-$C_4)$Alkylthio, $(C_2$-$C_4)$Alkenyl, $(C_2$-$C_4)$Alkinyl, $(C_2$-$C_4)$Alkenyloxy, $(C_2$-$C_4)$Alkinyloxy, Hydroxy, Amino, Acylamino, Mono- und Dialkylamino, Nitro, Carboxy, Cyano, Azido, $[(C_1$-$C_4)$Alkoxy]-carbonyl, $[(C_1$-$C_4)$Alkyl]-carbonyl, Formyl, Carbamoyl, Mono- und Di-$[(C_1$-$C_4)$alkyl]-aminocarbonyl, $(C_1$-$C_4)$Alkylsulfinyl, $(C_1$-$C_4)$Haloalkylsulfinyl, $(C_1$-$C_4)$Alkylsulfonyl, $(C_1$-$C_4)$Haloalkylsulfonyl und, im Falle cyclischer Reste, auch $(C_1$-$C_4)$Alkyl und $(C_1$-$C_4)$Haloalkyl substituiert ist,
oder einen Acylrest,

$R^5$ Wasserstoff, Halogen oder einen Rest der Formel $-B^1-Y^1$, wobei $B^1$ und $Y^1$ wie unten definiert sind,

A einen divalenten Rest der Formel $A^1$, $A^2$, $A^3$ oder $A^4$

$-CR^8R^9-CR^{10}R^{11}-$ $(A^2)$

$-CR^{12} = CR^{13}-$ $(A^3)$

$-CR^{14}R^{15}-CR^{16}R^{17}-CR^{18}R^{19}-$ $(A^4)$

$R^8, R^9, R^{10}, R^{11}, R^{12}, R^{13}, R^{14}, R^{15}, R^{16}, R^{17}, R^{18}, R^{19}$ jeweils unabhängig voneinander Halogen, Nitro, Cyano, Thiocyanato oder einen Rest der Formel $-B^2-Y^2$,

$(X)_n$ n Substituenten X und dabei X jeweils unabhängig voneinander Halogen, OH, $NH_2$, $NO_2$, CHO, COOH, CN, SCN oder einen Rest der Formel $-B^0-R$, wobei $B^0$ wie unten definiert ist und R $(C_1-C_6)$Alkyl, $(C_2-C_6)$Alkenyl oder $(C_2-C_6)$Alkinyl, wobei jeder der 3 letztgenannten Reste unsubstituiert oder substituiert ist, bedeutet, oder einen Rest der Formel $-B^0-R^0$, wobei $B^0$ wie unten definiert ist und $R^0$ $(C_3-C_6)$Cycloalkyl, $(C_3-C_6)$Cycloalkenyl oder Phenyl bedeutet, wobei jeder der letztgenannten drei Reste unsubstituiert oder substituiert ist, oder

zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, $(C_1-C_4)$Alkyl und Oxo substituiert ist,

n 0, 1, 2, 3 oder 4,

$B^0$,$B^1$,$B^2$ jeweils unabhängig voneinander eine direkte Bindung oder eine divalente Gruppe der Formel -O-, -S-, -CO-, -O-CO-, -CO-O-, -S-CO-, -CO-S-, -O-CO-O-, -NR'-, -NR'-CO-, -CO-NR'-, -O-CO-NR'-, -NR'-CO-O- oder -NR'-CONR''-, wobei R' und R'' unabhängig voneinander H oder $(C_1-C_4)$Alkyl sind,

$Y^1$,$Y^2$ jeweils unabhängig voneinander H oder einen acyclischen Kohlenwasserstoffrest mit 1 bis 6 C-Atomen, einen cyclischen Kohlenwasserstoffrest mit 3 bis 6 C-Atomen oder einen heterocyclischen Rest mit 3 bis 9 Ringatomen und 1 bis 3 Heteroringatomen aus der Gruppe N, O und S, wobei jeder der drei letztgenannten Reste unsubstituiert oder durch einen oder mehrere Reste aus der Gruppe Halogen, $(C_1-C_4)$Alkoxy, $(C_1-C_4)$Haloalkoxy, $(C_1-C_4)$Alkylthio, $(C_2-C_4)$Alkenyl, $(C_2-C_4)$Alkinyl, $(C_2-C_4)$Alkenyloxy, $(C_2-C_4)$Alkinyloxy, Hydroxy, Amino, Acylamino, Mono- und Dialkylamino, Nitro, Carboxy, Cyano, Azido, $[(C_1-C_4)$Alkoxy]-carbonyl, $[(C_1-C_4)$Alkyl]-carbonyl, Formyl, Carbamoyl, Mono- und Di-$[(C_1-C_4)$alkyl]-aminocarbonyl, $(C_1-C_4)$Alkylsulfinyl, $(C_1-C_4)$Haloalkylsulfinyl, $(C_1-C_4)$Alkylsulfonyl, $(C_1-C_4)$Haloalkylsulfonyl und, im Falle cyclischer Reste, auch $(C_1-C_4)$Alkyl und $(C_1-C_4)$Haloalkyl substituiert ist,

bedeuten.

Die Verbindungen der Formel (I) können durch Anlagerung einer geeigneten anorganischen oder organischen Säure, wie beispielsweise HCl, HBr, $H_2SO_4$ oder $HNO_3$, aber auch Oxalsäure oder Sulfonsäuren an eine basische Gruppe, wie z.B. Amino oder Alkylamino, Salze bilden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden. Salze können ebenfalls dadurch gebildet werden, daß bei geeigneten Substituenten, wie z.B. Sulfonsäuren oder Carbonsäuren, der Wasserstoff durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze oder Salze mit organischen Aminen.

In Formel (I) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Cycloalkyl bedeutet ein carbocyclisches, gesättigtes Ringsystem mit vorzugsweise 3-8 C-Atomen, z.B. Cyclopropyl, Cyclopentyl oder Cyclohexyl.

Halogen bedeutet beispielsweise Fluor, Chlor, Brom oder Iod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. Monohaloalkyl (= Monohalogenalkyl), Perhaloalkyl, $CF_3$, $CHF_2$, $CH_2F$, $CF_3CF_2$, $CH_2FCHCl$, $CCl_3$, $CHCl_2$, $CH_2CH_2Cl$; Haloalkoxy ist z.B. $OCF_3$, $OCHF_2$, $OCH_2F$, $CF_3CF_2O$, $OCH_2CF_3$ und $OCH_2CH_2Cl$; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl; Aryl bedeutet dabei ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl;
vorzugsweise bedeutet ein Kohlenwasserstoffrest Alkyl, Alkenyl oder Alkinyl mit bis zu 12 C-Atomen oder Cycloalkyl mit 3, 4, 5, 6 oder 7 Ringatomen oder Phenyl; entsprechendes gilt für einen Kohlenwasserstoffrest in einem Kohlenwasserstoffoxyrest.

Ein heterocyclischer Rest oder Ring (Heterocyclyl) kann gesättigt, ungesättigt oder heteroaromatisch sein; er enthält vorzugsweise ein oder mehrere Heteroeinheiten im Ring, d.h. Heteroatome oder Ringglieder, welche auch substituierte Heteroatome einschließen, vorzugsweise aus der Gruppe N, O, S, SO, $SO_2$; vorzugsweise ist er ein aliphatischer Heterocyclylrest mit 3 bis 7 Ringatomen oder ein heteroaromatischer Rest mit 5 oder 6 Ringatomen und enthält 1, 2 oder 3 Heteroeinheiten. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, oder ist ein partiell oder vollständig hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heterocyclyl oder Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, $(C_1\text{-}C_4)$Alkyl, vorzugsweise Methyl oder Ethyl, $(C_1\text{-}C_4)$Haloalkyl, vorzugsweise Trifluormethyl, $(C_1\text{-}C_4)$Alkoxy, vorzugsweise Methoxy oder Ethoxy, $(C_1\text{-}C_4)$Haloalkoxy, Nitro und Cyano. Besonders bevorzugt sind dabei die Substituenten Methyl, Methoxy und Chlor.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise $(C_1\text{-}C_4)$ Alkanoyl. Entsprechenes gilt für substituiertes Hydroxylamino oder Hydrazino.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, $(C_1\text{-}C_4)$Alkyl, $(C_1\text{-}C_4)$ Alkoxy, $(C_1\text{-}C_4)$Halogenalkyl, $(C_1\text{-}C_4)$Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Ein Acylrest bedeutet den Rest einer organischen Säure. Acyl bedeutet Formyl, $[(C_1\text{-}C_4)$Alkyl]-carbonyl, insbesondere

Phenylcarbonyl, Alkyloxycarbonyl, Phenyloxycarbonyl, Benzyloxycarbonyl, Alkylsulfonyl, Alkylsulfinyl und N-Alkyl-1-iminoalkyl. Dabei können die Reste jeweils im Alkyl- oder Phenylteil noch weiter substituiert sein, insbesondere im Alkylteil durch ein oder mehrere Reste aus der Gruppe Halogen, Alkoxy, Phenyl und Phenoxy. Substituenten im Phenylteil sind die bereits weiter oben allgemein für substituiertes Phenyl erwähnten Substituenten.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-Isomere sind alle von der Formel

(I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Vor allem aus den Gründen der höheren herbiziden Wirkung, besseren Selektivität und/oder besseren Herstellbarkeit sind erfindungsgemäße Verbindungen der genannten Formel (I) oder deren Salze von besonderem Interesse.

Von besonderem Interesse sind weiterhin erfindungsgemäße Verbindungen der Formel (I) und deren Salze, worin

$R^1$ $(C_1-C_4)$Alkyl,
das unsubstituiert oder durch einen oder mehrere Reste aus der Gruppe Halogen, $(C_1-C_4)$Alkoxy, $(C_1-C_4)$Alkylthio, $(C_3-C_6)$Cycloalkyl, das unsubstituiert oder durch einen oder mehrere Reste aus der Gruppe Halogen, $(C_1-C_4)$Alkyl, $(C_1-C_4)$Haloalkyl, $(C_1-C_4)$-Alkoxy und $(C_1-C_4)$Hatoalkoxy substituiert ist, und Phenyl, das unsubstituiert oder durch einen oder mehrere Reste aus der Gruppe Halogen, $(C_1-C_4)$Alkyl, $(C_1-C_4)$Haloalkyl, $(C_1-C_4)$Alkoxy, $(C_1-C_4)$Hatoalkoxy, $(C_1-C_4)$Alkylthio, Nitro, Cyano, $[(C_1-C_2)$Alkyl]carbonyl, Formyl, Carbamoyl, Mono- und Di-$[(C_1-C_2)$alkyl]aminocarbonyl und $(C_1-C_4)$Alkylsulfonyl substituiert ist, substituiert ist, oder
Phenyl, das unsubstituiert oder durch einen oder mehrere Reste aus der Gruppe Halogen, $(C_1-C_4)$Alkyl, $(C_1-C_4)$Haloalkyl, $(C_1-C_4)$Alkoxy, $(C_1-C_4)$Haloalkoxy, $(C_1-C_4)$Alkylthio, Nitro, Cyano, $[(C_1-C_2)$Alkyl]carbonyl, Formyl, Carbamoyl, Mono- und Di-$[(C_1-C_2)$alkyl]aminocarbonyl und $(C_1-C_4)$Alkylsulfonyl

substituiert ist,

$R^2$ und $R^3$ unabhängig voneinander Wasserstoff, Amino, Formyl, $(C_1-C_4)$Alkyl, Cyano-$(C_1-C_4)$alkyl, $(C_1-C_4)$Alkylamino, Di-$[(C_1-C_4)$alkyl]-amino, Halo-$(C_1-C_4)$alkyl, Hydroxy-$(C_1-C_4)$alkyl, $(C_1-C_4)$Alkoxy-$(C_1-C_4)$alkyl, Halo$(C_1-C_4)$alkoxy-$(C_1-C_4)$alkyl, $(C_2-C_6)$Alkenyl, Halo-$(C_2-C_6)$Alkenyl, $(C_2-C_6)$Alkinyl, Halo-$(C_2-C_6)$alkinyl, $(C_1-C_4)$Alkylamino-$(C_1-C_4)$alkyl, Di-$[(C_1-C_4)$alkyl]-amino-$(C_1-C_4)$alkyl, $(C_3-C_9)$Cycloalkylamino-$(C_1-C_4)$alkyl, $(C_3-C_9)$Cycloalkyl, $(C_3-C_9)$Heterocyclyl-$(C_1-C_4)$alkyl mit 3 bis 9 Ringgliedern, wobei die cyclischen Gruppen in den letztgenannten 3 Resten unsubstituiert oder durch einen oder mehrere Reste, vorzugsweise bis zu drei Reste, aus der Gruppe $(C_1-C_4)$Alkyl, Halogen und Cyano substituiert sind, oder
Phenyl, Phenoxy, Phenylcarbonyl, Phenylcarbonyl-$(C_1-C_4)$alkyl, $(C_1-C_4)$Alkoxy-carbonyl-$(C_1-C_4)$alkyl, $(C_1-C_4)$Alkylamino-carbonyl-$(C_1-C_4)$alkyl, $(C_1-C_4)$Alkyl-carbonyl, $(C_1-C_4)$Alkoxy-carbonyl, Aminocarbonyl, $(C_1-C_4)$Alkylamino-carbonyl, Phenoxy-$(C_1-C_4)$alkyl, Phenyl-$(C_1-C_4)$alkyl, Heterocyclyl, Heterocyclylamino, Heterocyclyloxy, Heterocyclylthio, oder einen der letztgenannten 16 Reste, der im acyclischen Teil oder, vorzugsweise, im cyclischen Teil durch einen oder mehrere Reste aus der Gruppe Halogen, Nitro, Cyano, $(C_1-C_4)$Alkyl, $(C_1-C_4)$Alkoxy, $(C_1-C_4)$Alkylthio, $(C_1-C_4)$Haloalkyl, $(C_1-C_4)$Haloalkoxy, Formyl, $(C_1-C_4)$Alkylcarbonyl, $(C_1-C_4)$Alkoxy-carbonyl, $(C_1-C_4)$Alkoxy, substituiert ist, wobei Heterocyclyl in den Resten jeweils 3 bis 9 Ringatome und 1 bis 3 Heteroringatome aus der Gruppe N, O und S enthält, oder
$R^2$ und $R^3$ gemeinsam mit dem Stickstoffatom der Gruppe $NR^2R^3$ einen heterocyclischen Rest mit 3 bis 6 Ringatomen und 1 bis 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, $(C_1-C_4)$Alkyl und Oxo substituiert ist,

$R^4$ Wasserstoff, Amino, Formyl, $(C_1-C_4)$Alkyl, Cyano-$(C_1-C_4)$alkyl, $(C_1-C_4)$Alkylamino, Di-$[(C_1-C_4)$alkyl]-amino, Ha-

lo-($C_1$-$C_4$)alkyl, Hydroxy-($C_1$-$C_4$)alkyl, ($C_1$-$C_4$)Alkoxy-($C_1$-$C_4$)alkyl, Halo($C_1$-$C_4$)alkoxy-($C_1$-$C_4$)alkyl, ($C_2$-$C_6$) Alkenyl, Halo-($C_2$-$C_6$)Alkenyl, ($C_2$-$C_6$)Alkinyl, Halo-($C_2$-$C_6$) alkinyl, ($C_1$-$C_4$)Alkylamino-($C_1$-$C_4$)alkyl, Di-[($C_1$-$C_4$)alkyl]-amino-($C_1$-$C_4$)alkyl, ($C_3$-$C_9$)Cycloalkylamino-($C_1$-$C_4$)alkyl, ($C_3$-$C_9$)Cycloalkyl, ($C_3$-$C_9$)Heterocyclyl-($C_1$-$C_4$)alkyl mit 3 bis 9 Ringgliedern, wobei die cyclischen Gruppen in den letztgenannten 3 Resten unsubstituiert oder durch einen oder mehrere Reste, vorzugsweise bis zu drei Reste, aus der Gruppe ($C_1$-$C_4$)Alkyl, Halogen und Cyano substituiert sind, oder

Phenyl, Phenoxy, Phenylcarbonyl, Phenylcarbonyl-($C_1$-$C_4$) alkyl, ($C_1$-$C_4$)Alkoxy-carbonyl-($C_1$-$C_4$)alkyl, ($C_1$-$C_4$)Alkylamino-carbonyl-($C_1$-$C_4$)alkyl, ($C_1$-$C_4$)Alkyl-carbonyl, ($C_1$-$C_4$)Alkoxy-carbonyl, Aminocarbonyl, ($C_1$-$C_4$)Alkylamino-carbonyl, Phenoxy-($C_1$-$C_4$)alkyl, Phenyl-($C_1$-$C_4$)alkyl, Heterocyclyl, Heterocyclylamino, Heterocyclyloxy, Heterocyclylthio, oder einen der letztgenannten 16 Reste, der im acyclischen Teil oder, vorzugsweise, im cyclischen Teil durch einen oder mehrere Reste aus der Gruppe Halogen, Nitro, Cyano, ($C_1$-$C_4$)Alkyl, ($C_1$-$C_4$)Alkoxy, ($C_1$-$C_4$)Alkylthlo, ($C_1$-$C_4$)Haloalkyl, ($C_1$-$C_4$)Haloalkoxy, Formyl, ($C_1$-$C_4$)Alkylcarbonyl, ($C_1$-$C_4$)Alkoxy-carbonyl, ($C_1$-$C_4$)Alkoxy, substituiert ist, wobei Heterocyclyl in den Resten jeweils 3 bis 9 Ringatome und 1 bis 3 Heteroringatome aus der Gruppe N, O und S enthält,

$R^5$ Wasserstoff, ($C_1$-$C_4$)Alkyl, ($C_1$-$C_4$)Halogenalkyl oder ($C_3$-$C_6$)Cycloalkyl,

A einen divalenten Rest der genannten Formel $A^1$, $A^2$, $A^3$ oder $A^4$,

$R^6$,$R^7$,$R^8$,$R^9$,$R^{10}$,$R^{11}$,$R^{12}$,$R^{13}$,$R^{14}$,$R^{15}$,$R^{16}$ $R^{17}$,$R^{18}$,$R^{19}$ jeweils unabhängig voneinander Wasserstoff, Halogen, ($C_1$-$C_8$)Alkyl, ($C_2$-$C_6$)Alkenyl, ($C_2$-$C_6$)Alkinyl, ($C_3$-$C_9$)Cycloalkyl, ($C_3$-$C_9$)Cycloalkenyl, Phenyl, Heterocyclyl mit 3 bis 6 Ringatomen und 1 bis 3 Heteroringatomen aus der Gruppe N, O und S, wobei jeder der letztgenannten 7 Reste unsubstituiert oder durch einen oder mehrere Reste aus der Gruppe Halogen, ($C_1$-$C_4$)Alkoxy, ($C_1$-$C_4$)Haloalkoxy, ($C_1$-$C_4$)Alkylthio, Amino, Mono- und Di[($C_1$-$C_4$)alkyl]amino, ($C_1$-$C_4$) Alkanoylamino, Benzoylamino, Nitro, Cyano, [($C_1$-$C_4$)Alkyl] carbonyl, Formyl, Carbamoyl, Mono- und Di-[($C_1$-$C_4$)alkyl] aminocarbonyl, ($C_1$-$C_4$)Alkylsulfonyl und, im Fall cyclischer Reste, auch ($C_1$-$C_4$)Alkyl und ($C_1$-$C_4$)Haloalkyl substituiert ist,

$(X)_n$ n Substituenten X und dabei X jeweils unabhängig voneinander Halogen, Hydroxy, Amino, Nitro, Formyl, Carboxy, Cyano, Thiocyanato, ($C_1$-$C_4$)Alkyl, Cyano-($C_1$-$C_4$)alkyl, ($C_1$-$C_4$)Alkoxy, ($C_1$-$C_4$)Alkylamino, Di-[($C_1$-$C_4$)alkyl]-amino, Halo-($C_1$-$C_4$)alkyl, Hydroxy-($C_1$-$C_4$)alkyl, ($C_1$-$C_4$)Alkoxy-($C_1$-$C_4$)alkyl, Halo($C_1$-$C_4$)alkoxy-($C_1$-$C_4$)alkyl, ($C_1$-$C_4$) Alkylthio, Halo-($C_1$-$C_4$)alkylthio, ($C_2$-$C_6$)Alkenyl, Halo-($C_2$-$C_6$)Alkenyl, ($C_2$-$C_6$)Alkinyl, Halo-($C_2$-$C_6$)alkinyl, ($C_1$-$C_4$)Alkylamino-($C_1$-$C_4$)alkyl, Di-[($C_1$-$C_4$)alkyl]-amino-($C_1$-$C_4$)alkyl, ($C_3$-$C_9$)Cycloalkylamino-($C_1$-$C_4$)alkyl, ($C_3$-$C_9$)Cycloalkyl, Heterocyclyl-($C_1$-$C_4$)alkyl mit 3 bis 9 Ringgliedern, wobei die cyclischen Gruppen in den letztgenannten 3 Resten unsubstituiert oder durch einen oder mehrere Reste, vorzugsweise bis zu drei Reste, aus der Gruppe ($C_1$-$C_4$)Alkyl, Halogen und Cyano substituiert sind,

oder Phenyl, Phenoxy, Phenylcarbonyl, Phenylcarbonyl-($C_1$-$C_4$)alkyl, ($C_1$-$C_4$)Alkoxy-carbonyl-($C_1$-$C_4$)alkyl, ($C_1$-$C_4$)Alkylamino-carbonyl-($C_1$-$C_4$)alkyl, ($C_1$-$C_4$)Alkyl-carbonyl, ($C_1$-$C_4$)Alkoxy-carbonyl, Aminocarbonyl, ($C_1$-$C_4$)Alkylamino-carbonyl, Phenoxy-($C_1$-$C_4$)alkyl, Phenyl-($C_1$-$C_4$)alkyl, Heterocyclyl, Heterocyclylamino, Heterocyclyloxy, Heterocyclylthio oder einen der letztgenannten 16 Reste, der im acyclischen Teil oder, vorzugsweise, im cyclischen Teil durch einen oder mehrere Reste aus der Gruppe Halogen, Nitro, Cyano, ($C_1$-$C_4$)Alkyl, ($C_1$-$C_4$)Alkoxy, ($C_1$-$C_4$)Alkylthio, ($C_1$-$C_4$)Haloalkyl, ($C_1$-$C_4$)Haloalkoxy, Formyl, ($C_1$-$C_4$)Alkyl-carbonyl, ($C_1$-$C_4$)Alkoxy-carbonyl, ($C_1$-$C_4$)Alkoxy, substituiert ist, wobei Heterocyclyl in den Resten jeweils 3 bis 9 Ringatome und 1 bis 3 Heteroringatome aus der Gruppe N, O und S enthält, oder

zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, ($C_1$-$C_4$)Alkyl und Oxo substituiert ist,

bedeuten.

Von besonderem Interesse sind weiterhin erfindungsgemäße Verbindungen der Formel (I) und deren Salze, worin

| | |
|---|---|
| $R^1$ | ($C_1$-$C_4$)Alkyl, ($C_1$-$C_4$)Haloalkyl, Benzyl oder [($C_3$-$C_6$)Cycloalkyl]-($C_1$-$C_2$)alkyl, |
| $R^2$ und $R^3$ | unabhängig voneinander Wasserstoff, Amino, Formyl, ($C_1$-$C_4$)Alkyl, ($C_1$-$C_4$)Alkoxy-($C_1$-$C_4$)alkyl, ($C_2$-$C_6$)Alkenyl, ($C_2$-$C_6$)Alkinyl, ($C_1$-$C_4$)Alkylamino-($C_1$-$C_4$)alkyl, Di-[($C_1$-$C_4$)alkyl]-amino-($C_1$-$C_4$)alkyl oder Phenyl, Phenyl-($C_1$-$C_4$)alkyl oder Phenoxy-carbonyl oder einen der letztgenannten drei Reste, der im Phenylteil bis zu dreifach durch Reste aus der Gruppe Halogen, ($C_1$-$C_4$)Alkyl, ($C_1$-$C_4$)Alkoxy und ($C_1$-$C_4$)Alkoxy-carbonyl substituiert ist, oder<br>$R^2$ und $R^3$ gemeinsam mit dem Stickstoffatom der Gruppe $NR^2R^3$ einen heterocyclischen Rest mit 3 bis 6 Ringatomen und 1 bis 2 Heteroringatomen, wobei neben dem N-Atom das gegebenenfalls weitere Heteroringatom aus der Gruppe N und O ausgewählt ist und der Rest unsubstituiert oder durch einen oder mehrere Reste aus der Gruppe Halogen, ($C_1$-$C_4$)Alkyl und Oxo substituiert ist, |
| $R^4$ | Wasserstoff, Amino, Formyl, ($C_1$-$C_4$)Alkyl, Di-[($C_1$-$C_4$)alkyl]-amino, ($C_1$-$C_4$)Alkoxy-($C_1$-$C_4$)alkyl, ($C_2$-$C_6$)Alkenyl, ($C_2$-$C_6$)Alkinyl, ($C_1$-$C_4$)Dialkylamino-($C_1$-$C_4$)alkyl, Phenyl, Phenoxy-($C_1$-$C_4$)alkyl, Phenyl-($C_1$-$C_4$)alkyl, Phenoxy-carbonyl, Phenylamino-carbonyl oder einen der letztgenannten fünf Reste, der im Phenylteil einfach bis dreifach durch Reste aus der Gruppe Halogen, ($C_1$-$C_4$)Alkyl, ($C_1$-$C_4$)Alkoxy und ($C_1$-$C_4$)Alkoxy-carbonyl substituiert ist, |
| $R^5$ | Wasserstoff, ($C_1$-$C_4$)Alkyl, ($C_1$-$C_4$)Halogenalkyl oder ($C_3$-$C_6$)Cycloalkyl, |
| A | einen divalenten Rest der genannten Formel $A^1$, $A^2$, $A^3$ oder $A^4$, |
| $R^6$,$R^7$,$R^8$,$R^9$,$R^{10}$,$R^{11}$,$R^{12}$,$R^{13}$,$R^{14}$,$R^{15}$,$R^{16}$,$R^{17}$,$R^{18}$,$R^{19}$ | jeweils unabhängig voneinander Wasserstoff, Halogen oder ($C_1$-$C_4$)Alkyl, vorzugsweise H oder ($C_1$-$C_4$)Alkyl, |
| $(X)_n$ | n Substituenten X und dabei X jeweils unabhängig vonein- |

ander Halogen, Hydroxy, $(C_1-C_4)$Alkyl oder $(C_1-C_4)$Alkoxy

bedeuten.

Bevorzugte erfindungsgemäße Verbindungen der Formel (I) und deren Salze sind solche, worin

| | |
|---|---|
| $R^1$ | $(C_1-C_4)$Alkyl, $(C_1-C_4)$Haloalkyl oder $[(C_3-C_6)$Cycloalkyl]-methyl, vorzugsweise $-CH_3$, $-CH_2F$, $-CHF_2$, $-CF_3$, $-CH_2Cl$, $-CHCl_2$, $-CCl_3$, $-CH_2Br$, $-CHBr_2$, $-CH_2CH_3$, $-CH_2CH_2F$, $-CF_2CHF_2$, $-CH_2CH_2Cl$, $-CH_2CH_2Br$, $-CH(CH_3)_2$, $-CF(CH_3)_2$, $-C(CH_3)_2Cl$, $-CH_2CH_2CH_2F$, $-CH_2CH_2CH_2Cl$ der Cyclopropylmethyl, |
| | $R^2$ und $R^3$ unabhängig voneinander Wasserstoff, Amino, Formyl oder $(C_1-C_4)$Alkyl oder |
| | $R^2$ und $R^3$ gemeinsam mit dem Stickstoffatom der Gruppe $NR^2R^3$ einen heterocyclischen Rest mit 4 bis 6 Ringatomen und 1 bis 2 Heteroringatomen, wobei neben dem N-Atom das gegebenenfalls weitere Heteroringatom aus der Gruppe N und O ausgewählt ist, |
| $R^4$ | Wasserstoff oder $(C_1-C_4)$Alkyl, |
| $R^5$ | H, $(C_1-C_4)$Alkyl oder $(C_1-C_4)$Haloalkyl, vorzugsweise H, $CH_3$, $C_2H_5$, n- oder i-$C_3H_7$, n-Butyl, $CF_3$ oder $CH_2CF_3$, insbesondere $CH_3$, |
| A | einen divalenten Rest der genannten Formel $A^1$, $A^2$ oder $A^3$, |
| $R^6,R^7,R^8,R^9,R^{10},R^{11},R^{12},R^{13}$ | jeweils unabhängig voneinander Wasserstoff oder $(C_1-C_4)$Alkyl, |
| $(X)_n$ | n Substituenten X und dabei X jeweils unabhängig voneinander Halogen, Hydroxy, $(C_1-C_4)$Alkyl oder $(C_1-C_4)$Alkoxy |

bedeuten.

Bevorzugt sind auch erfindungsgemäße Verbindungen der Formel (I) und deren Salze, die ein oder mehrere der Merkmale aus den obengenannten bevorzugten Verbindungen enthalten.

Gegenstand der vorliegenden Erfindung sind auch Verfahren zur Herstellung der Verbindungen der allgemeinen Formel (I) oder deren Salze, dadurch gekennzeichnet, daß man

a) eine Verbindung der Formel (II),

$$R^1 - Fu \qquad (II)$$

worin Fu eine funktionelle Gruppe aus der Gruppe Carbonsäureester, Carbonsäureorthoester, Carbonsäurechlorid, Carbonsäureamid, Carbonsäureanhydrid und Trichlormethyl bedeutet,
mit einem Biguanidid der Formel (III) oder einem Säureadditionssalz hiervon

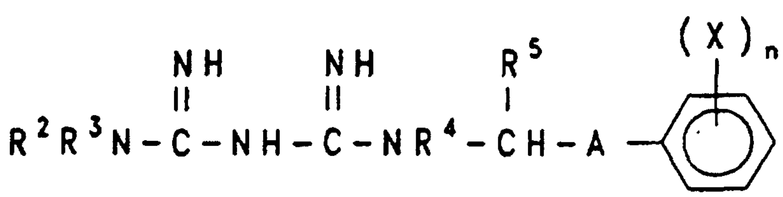

(III)

umsetzt oder

b) eine Verbindung der Formel (IV),

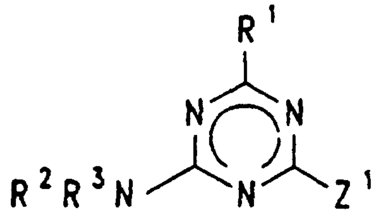

(IV)

worin $Z^1$ einen austauschfähigen Rest oder eine Abgangsgruppe, z.B. Chlor, Trichlormethyl, $(C_1-C_4)$Alkylsulfonyl und unsubstituiertes oder substituiertes Phenyl-$(C_1-C_4)$alkylsulfonyl oder $(C_1-C_4)$Alkylphenylsulfonyl, bedeutet, mit einem geeigneten Amin der Formel (V) oder einem Säureadditionssalz hiervon

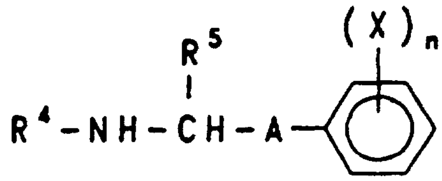

(V)

umsetzt,

wobei in den Formeln (II), (III), (IV) und (V) die Reste $R^1$, $R^2$, $R^3$, $R^4$, $R^5$, A und X sowie n wie in Formel (I) definiert sind.

Die Umsetzung der Verbindungen der Formeln (II) und (III) erfolgt vorzugsweise basenkatalysiert in einem inerten organischen Lösungsmittel, wie z.B. Tetrahydrofuran (THF), Dioxan, Acetonitril, Dimethylformamid (DMF), Methanol und Ethanol, bei Temperaturen zwischen -10 °C und dem Siedepunkt des Lösungsmittel, vorzugsweise bei 20 °C bis 60 °C; falls Säureadditionssalze der Formel (III) verwendet werden, setzt man diese in der Regel mit Hilfe einer Base in situ frei. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicylco[5.4.0]undec-7-en (DBU). Die jeweilige Base wird dabei beispielsweise im Bereich von 0,1 bis 3 Moläquivalenten bezogen auf die Verbindung der Formel (III) eingesetzt. Die Verbindung der Formel (II) kann im Verhältnis zur Verbindung der Formel (III) beispielsweise äquimolar oder mit bis zu 2 Moläquvälenten Überschuß eingesetzt werden. Grundsätzlich sind die entsprechenden Verfahren in der Literatur bekannt (vergleiche: Comprehensive Heterocyclic Chemistry, A.R. Katritzky, C.W. Rees, Pergamon Press, Oxford, New York, 1984, Vol.3; Part 2B; ISBN 0-08-030703-5, S.290).

Die Umsetzung der Verbindungen der Formeln (IV) und (V) erfolgt vorzugsweise basenkatalysiert in einem inerten organischen Lösungsmittel, wie z.B. THF, Dioxan, Acetonitril, DMF, Methanol und Ethanol, bei Temperaturen zwischen -10 °C und dem Siedepunkt des jeweiligen Lösungsmittels oder Lösungsmittelgemisches, vorzugsweise bei 20 °C bis 60 °C, wobei die Verbindung (V), falls als Säureadditionssalz eingesetzt, gegebenenfalls in situ mit einer Base freigesetzt wird. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei in der Regel im Bereich von 1 bis 3 Moläquivalenten bezogen auf die Verbindung der Formel (IV) eingesetzt, die Verbindung der Formel (IV) kann beispielsweise äquimolar zur Verbindung der Formel (V) oder mit bis zu 2 Moläquivalenten Überschuß eingesetzt werden. Grundsätzlich sind die entsprechenden Verfahren aus der Literatur bekannt (vgl. Comprehensive Heterocyclic Chemistry, A.R. Katritzky, C.W. Rees, Pergamon Press, Oxford, New York, 1984, Vol.3; Part 2B; ISBN 0-08-030703-5, S. 482).

Die Edukte der Formeln (II), (III), (IV) und (V) sind entweder kommerziell erhältlich oder können nach oder analog literaturbekannten Verfahren hergestellt werden. Die Verbindungen können beispielsweise auch nach einem der nachfolgend beschriebenen Verfahren hergestellt werden.

Die Verbindung der Formel (IV), oder eine direkte Vorstufe davon, läßt sich beispielweise wie folgt herstellen:

1. Durch Reaktion einer Verbindung der Formel (II) mit einem Amidino-thioharnstoff-Derivat der Formel (VI),

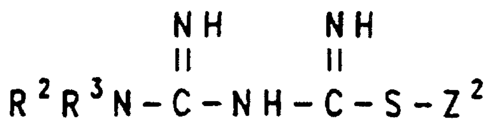 (VI)

worin $Z^2$ ($C_1$-$C_4$)-Alkyl oder Phenyl-($C_1$-$C_4$)-alkyl bedeutet und $R^2$ und $R^3$ wie in Formel (I) definiert sind, werden Verbindungen der Formel (IV) erhalten, in denen $Z^1$ = $-SZ^2$ bedeutet.

2. Durch Umsetzung eines Amidins der Formel (VII) oder eines Säureadditionssalzes davon,

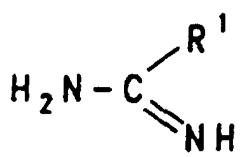 (VII)

worin $R^1$ wie in Formel (I) definiert ist,
mit einem N-Cyanodithioiminocarbonat der Formel (VIII),

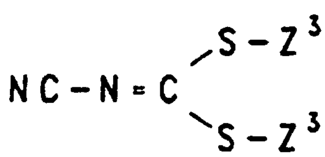 (VIII)

worin $Z^3$ ($C_1$-$C_4$)-Alkyl oder Phenyl-($C_1$-$C_4$)-alkyl bedeutet, werden Verbindungen der Formel (IV) erhalten, worin $Z^1$ = $-S-Z^3$ bedeutet.

3. Durch Umsetzung eines Alkali-dicyanamids mit einem Carbonsäurederivat der genannten Formel (II) werden Verbindungen der Formel (IV) erhalten, worin $Z^1$ = $NH_2$ bedeutet.

4. Durch Umsetzung von Trichloracetonitril mit einem Nitril der Formel (IX),

$$R^1 - CN \qquad (IX)$$

worin $R^1$ wie in Formel (I) definiert ist, werden zunächst Verbindungen der Formel (X),

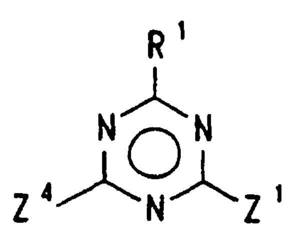 (X)

worin $Z^1$ und $Z^4$ jeweils $CCl_3$ bedeuten, erhalten, welche durch nachfolgende Umsetzung mit Verbindungen der Formel $HNR^2R^3$ ($R^2$ und $R^3$ wie in Formel (I)), zu Verbindungen der Formel (IV), worin $Z^1$ = $CCl_3$ bedeutet, führen.

Die Umsetzung der Carbonsäurederivate der Formel (II) mit den Amidinothioharnstoff-Derivaten der Formel (VI) erfolgt vorzugsweise basenkatalysiert in einem organischen Lösungsmittel, wie z.B. Aceton, THF, Dioxan, Acetonitril, DMF, Methanol, Ethanol, bei Temperaturen von -10 °C bis zum Siedepunkt des Lösungsmittel, vorzugsweise bei 0 °C bis 20 °C. Die Umsetzung kann aber auch in Wasser oder in wässrigen Lösungsmitttelgemischen mit einem oder mehreren der obengenannten organischen Lösungsmitteln erfolgen. Falls (VI) als Säureadditionssalz eingesetzt wird, kann es gegebenenfalls in situ mit einer Base freigesetzt werden. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicylco[5.4.0]undec-7-en (DBU). Die jeweilige Base wird dabei z.B. im Bereich von 1 bis 3 Moläquivalenten bezogen auf die Verbindung der Formel (VI) eingesetzt. Verbindungen der Formeln (II) und (VI) können beispielsweise äquimolar oder mit bis zu 2 Moläquivalenten Überschuß an Verbindung der Formel (II) eingesetzt werden. Grundsätzlich sind die entsprechenden Verfahren literaturbekannt (vergl.: H. Eilingsfeld, H. Scheuermann, Chem. Ber.; 1967, 100, 1874), die entsprechenden Zwischenprodukte der Formel (IV) sind neu.

Die Umsetzung der Amidine der Formel (VII) mit den N-Cyanodithioiminocarbonaten der Formel (VIII) erfolgt vorzugsweise basenkatalysiert in einem inerten organischen Lösungsmittel, wie z.B. Acetonitril, DMF, Dimethylacetamid (DMA), N-Methylpyrrolidon (NMP), Methanol und Ethanol, bei Temperaturen von -10 °C bis zum Siedepunkt des Lösungsmittels, vorzugsweise bei 20 °C bis 80 °C. Falls (VII) als Säureadditionssalz eingesetzt wird, kann es gegebenenfalls in situ mit einer Base freigesetzt werden. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei z. B. in Bereich von 1 bis 3 Moläquivalenten bezogen auf die Verbindung der Formel (VIII) eingesetzt, Verbindungen der Formel (VII) und (VIII) können in der Regel äquimolar oder mit 2 Moläquivalenten Überschuß an Verbindung der Formel (II) eingesetzt werden. Grundsätzlich sind die entsprechenden Verfahren literaturbekannt (vergl.: T.A. Riley, W.J. Henney, N.K. Dalley, B.E. Wilson, R.K. Robins; J. Heterocyclic Chem.; 1986, 23 (6), 1706-1714), die entsprechenden Zwischenprodukte der Formel (IV) sind neu.

Die Herstellung von Zwischenprodukten der Formel (X) mit $Z^1$ = Chlor kann durch Reaktion von Alkali-dicyanamid mit einem Carbonsäurederivat der Formel (II), wobei dann Fu bevorzugt die funktionelle Gruppe Carbonsäurechlorid oder Carbonsäureamid bedeutet, erfolgen. Die Umsetzung der Reaktionskomponenten erfolgt beispielsweise säurekatalysiert in einem inerten organischen Lösungsmittel wie z.B. Toluol, Chlorbenzol, chlorierten Kohlenwasserstoffen bei Temperaturen zwischen -10 °C und dem Siedepunkt des Lösungsmittels, vorzugsweise bei 20 °C bis 80 °C, wobei die entstehenden Intermediate in situ mit einem geeigneten Chlorierungsreagenz wie beispielsweise Phosphoroxychlorid chloriert werden können. Geeignete Säuren sind z.B. Halogenwasserstoffsäuren, wie HCl, oder auch Lewis-Säuren, wie z.B. $AlCl_3$ oder $BF_3$ (vergl. US-A-5095113, DuPont).

Die Herstellung von Zwischenprodukten der Formel (X) mit $Z^1$, $Z^4$ = Trihalogenmethyl kann durch Reaktion der entsprechenden Trihalogenessigsäurenitrile mit einem Carbonsäurenitril der Formel (IX) erfolgen. Die Umsetzung der Reaktionskomponenten erfolgt beispielsweise säurekatalysiert in einem inerten organischen Lösungsmittel wie z. B. Toluol, Chlorbenzol, chlorierten Kohlenwasserstoffen bei Temperaturen zwischen -40 °C und dem Siedepunkt des Lösungsmittels, vorzugsweise bei -10 °C bis 30 °C. Geeignete Säuren sind z.B. Halogenwasserstoffsäuren wie HCl oder auch Lewis-Säuren wie z.B. $AlCl_3$ oder $BF_3$ (vgl. EP-A-130939, Ciba Geigy).

Zwischenprodukte der Formel (IV), worin $Z^1$ = ($C_1$-$C_4$)Alkylmercapto oder unsubstituiertes Phenyl-($C_1$-$C_4$)-alkylmercapto ist, können in einem inerten organischen Lösungsmittel wie z .B. Toluol, Chlorbenzol, chlorierten Kohlenwasserstoffen oder anderen bei Temperaturen zwischen -40 °C und dem Siedepunkt des Lösungsmittel, vorzugsweise bei 20 °C bis 80 °C, mit einem geeigneten Chlorierungsreagenz wie z.B. elementarem Chlor oder Phosporoxychlorid zu reaktionsfähigeren Chlortriazinen der Formel (IV), worin $Z^1$ = Cl ist, überführt werden (vgl. J.K. Chakrabarti, D.E. Tupper; Tetrahedron 1975, 31(16), 1879-1882).

Zwischenprodukte der Formel (IV), wobei $Z^1$ = ($C_1$-$C_4$)Alkylmercapto oder unsubstituiertes oder substituiertes Phenyl-($C_1$-$C_4$)-alkylmercapto oder ($C_1$-$C_4$)Alkyl-phenylthio ist, können in einem geeigneten Lösungsmittel wie z.B. chlorierten Kohlenwasserstoffen, Essigsäure, Wasser, Alkoholen, Aceton oder Mischungen hiervon bei Temperaturen zwischen 0 °C und dem Siedepunkt des Lösungsmittels, vorzugsweise von 20 °C bis 80 °C, mit einem geeigneten Oxidationssreagenz wie z.B. m-Chlorperbenzoesäure, Wasserstoffperoxid, Kaliumperoxomonosulfat oxidiert werden (vergl.: T.A. Riley, W.J. Henney, N.K. Dalley, B.E. Wilson, R.K. Robins; J. Heterocyclic Chem.; 1986, 23 (6), 1706-1714).

Zur Herstellung der Säureadditionssalze der Verbindungen der Formel (I) kommen folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid oder Benzin und Hinzufügen der Säure bei Temperaturen von 0 bis 100 °C erhalten werden und in bekannter Weise, z.B. durch Abfiltrieren, isoliert und gegebenenfalls durch Waschen mit einem inerten organischen Lösemittel gereinigt werden.

Die Basenadditionssalze der Verbindungen der Formel (I) werden vorzugsweise in inerten polaren Lösungsmitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat, Kalium-tert.Butylat, oder Ammoniak oder Ethanolamin.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) und deren Salze, im folgenden zusammen als (erfindungsgemäße) Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberninaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. Weed Research 26, 441-445 (1986), oder "The Pesticide Manual", 10th edition, The British Crop Protection Council and the Royal Soc. of Chemistry, 1994 und dort zitierter Literatur beschrieben sind. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):

acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; buualin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazamethabenz-methyl; imazapyr; imazaquin und Salze wie das Ammoniumsalz; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluormethyl)-phenoxy]-2-naphthalenyl]-oxy]-propansäure und -methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thizopyr (Mon-13200); thidiazimin (SN-24085); thifensulfuron-methyl; thiobencarb; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

A. Chemische Beispiele

Beispiel A1

2-Amino-4-(1,1,2,2-tetrafluorethyl)-6-(1-phenyl-3-butylamino)-1,3,5-triazin (siehe Tabelle 1, Beispiel 55)

Zu 6,7 g (0,025 mol) 3-Biguanidino-1-phenyl-butan-hydrochlorid in 50 ml Methanol und 7 g gemahlenem Molekularsieb 3Ä fügt man eine aus 1,2 g (0,05 mol) Natrium und 100 ml Methanol hergestellte Methanolatlösung. Danach gibt man 7,2 g (0,045 mol) 1,1,2,2-Tetrafluorpropansäure-methylester hinzu und rührt 2 Stunden bei 25°C und dann 4 Stunden bei 65°C. Die Reaktionsmischung wird filtriert, das Filtrat eingeengt und der Rückstand in Essigester aufgenommen. Es wird mit Wasser gewaschen und mit Natriumsulfat getrocknet. Das Trockenmittel wird abfiltriert und das Lösungsmittel im Vakuum eingedampft. Nach Reinigung mit Säulenchromatographie (Laufmittel: Essigsäureethylester) erhält man 6,95 g (81 % d.Th.) 2-Amino-4-(1,1,2,2-tetrafluorethyl)-6-(1-phenyl-3-butylamino)-1,3,5-triazin.

Beispiel A2

2-Amino-4-isopropyl-6-[1-(4-fluorphenyl)-3-butylamino]-1,3,5-triazin (siehe Tabelle 1, Beispiel 2)

2,6 g (0,015 mol) 2-Amino-4-chlor-6-isopropyl-1,3,5-triazin und 2,1 g (0,015 mol) $K_2CO_3$ werden in 50 ml Acetonitril vorgelegt. Zu dieser Lösung tropft man 2,5 g (0,015 mol) 1-(4-Fluorphenyl)-3-butylamin, gelöst in 20 ml Acetonitril, hinzu. Anschließend läßt man 3 Stunden am Rückfluß kochen. Danach werden die festen Bestandteile abgesaugt und das Filtrat einrotiert. Der Rückstand wird mittels Säulenchromatographie (Laufmittel: Essigsäureethylester) gereinigt. Ausbeute: 4,1 g (90 % d.Th.).

Die in den nachfolgenden Tabellen 1 und 2 beschriebenen Verbindungen erhält man gemäß oder analog zu den vorstehenden Beispielen A1 und A2. In den Tabellen bedeuten:

| | |
|---|---|
| Nr. = | Beispiel oder Beispielnummer |
| Phys. Daten = | Charakteristische physikalische Daten der Verbindung |
| Me = | Methyl |
| Et = | Ethyl |
| Pr = | Propyl |
| i-Pr = | Isopropyl |
| c-Pr = | Cyclopropyl |
| t-Bu = | tertiär-Butyl |
| Ph = | Phenyl |
| $(X)_n$ = | Position und Art des Substituenten am Phenylring (Position 1 = Bindung zu A);<br>"-" = kein Substituent am Phenylring (n = 0) |

Tabelle 1: Verbindungen der Formel (I)

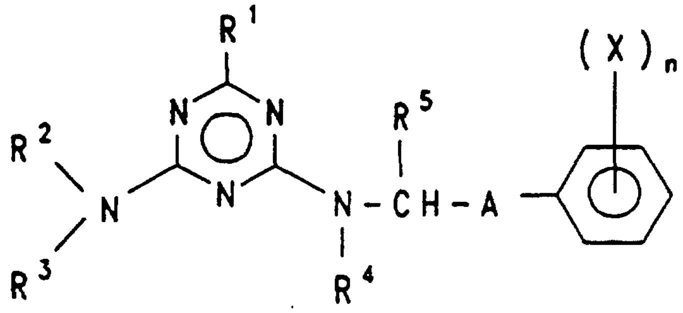

(I)

| Nr. | $R^1$ | $R^2$ | $R^3$ | $R^4$ | $R^5$ | A | $(X)_n$ | Phys. Daten |
|---|---|---|---|---|---|---|---|---|
| 1 | i-Pr | H | H | H | Me | $(CH_2)_2$ | - | NMR, siehe Ende Tabelle |
| 2 | i-Pr | H | H | H | Me | $(CH_2)_2$ | 4-F | NMR, siehe Ende Tabelle |
| 3 | i-Pr | H | H | H | Me | $(CH_2)_2$ | 2-Cl | NMR, siehe Ende Tabelle |
| 4 | i-Pr | H | H | H | Me | $(CH_2)_2$ | 3,5-$Me_2$ | NMR, siehe Ende Tabelle |
| 5 | i-Pr | H | H | H | Me | $(CH_2)_2$ | 4-(t-Bu) | Oel |
| 6 | i-Pr | H | H | H | Me | $(CH_2)_2$ | 3-J | Oel |
| 7 | i-Pr | H | H | H | Me | $(CH_2)_2$ | 2,5-$Me_2$ | |
| 8 | i-Pr | H | H | H | Me | $(CH_2)_2$ | 2,6-$Cl_2$ | |
| 9 | i-Pr | H | H | H | Me | $(CH_2)_2$ | 2,4,6-$Me_3$ | Oel |
| 10 | i-Pr | H | H | H | Me | $(CH_2)_2$ | 3,5-$F_2$ | |
| 11 | i-Pr | H | H | H | Me | $(CH_2)_2$ | 3-Me | Oel |
| 12 | i-Pr | H | H | H | Me | $(CH_2)_2$ | 3-$CF_3$ | |
| 13 | i-Pr | H | H | H | Me | $(CH_2)_2$ | 3-Br | |
| 14 | i-Pr | H | H | H | Me | $(CH_2)_2$ | 4-Me | |
| 15 | t-Bu | H | H | H | Me | $(CH_2)_2$ | 3,5-$Me_2$ | |
| 16 | Me | H | H | H | Me | $(CH_2)_2$ | 3,4,5-$Me_3$ | |
| 17 | Et | H | H | H | Me | $(CH_2)_2$ | 3,5-$Me_2$ | |
| 18 | $CH_2CH_2F$ | H | H | H | Me | $(CH_2)_2$ | 2,4,6-$Me_3$ | |
| 19 | $CH_2CH_2Cl$ | H | H | H | Me | $(CH_2)_2$ | 2-Me | |
| 20 | $CH_2CH_2Br$ | H | H | H | Me | $(CH_2)_2$ | 2-Cl | |
| 21 | $C(CH_3)_2Cl$ | H | H | H | Me | $(CH_2)_2$ | 2-Br | |
| 22 | $CH_2CH_2CH_2F$ | H | H | H | Me | $(CH_2)_2$ | 2-J | |
| 23 | $CH_2CH_2CH_2Cl$ | H | H | H | Me | $(CH_2)_2$ | 2-$CCl_3$ | |
| 24 | $CF_3$ | H | H | H | Me | $(CH_2)_2$ | - | |
| 25 | $CF_3$ | H | H | H | Me | $(CH_2)_2$ | 3,5-$F_2$ | |
| 26 | $CF_3$ | H | H | H | Me | $(CH_2)_2$ | 3,5-$Cl_2$ | |
| 27 | $CF_3$ | H | H | H | Me | $(CH_2)_2$ | 3,5-$Me_2$ | |
| 28 | $CF_3$ | H | H | H | Me | $(CH_2)_2$ | 3,5-$Br_2$ | |

| Nr. | $R^1$ | $R^2$ | $R^3$ | $R^4$ | $R^5$ | A | $(X)_n$ | Phys. Daten |
|---|---|---|---|---|---|---|---|---|
| 29 | $CH_2F$ | H | H | H | Me | $(CH_2)_2$ | 4-Me | |
| 30 | $CHF_2$ | H | H | H | Me | $(CH_2)_2$ | 4-Cl | |
| 31 | $CH_2Cl$ | H | H | H | Me | $(CH_2)_2$ | 3,5-$Me_2$ | |
| 32 | $CHCl_2$ | H | H | H | Me | $(CH_2)_2$ | 3-Me | |
| 33 | $CCl_3$ | H | H | H | Me | $(CH_2)_2$ | 3-Cl | |
| 34 | $CH_2Br$ | H | H | H | Me | $(CH_2)_2$ | 3-J | |
| 35 | $CHBr_2$ | H | H | H | Me | $(CH_2)_2$ | 3-J | |
| 36 | $CF(CH_3)_2$ | H | H | H | Me | $(CH_2)_2$ | 4-(i-Pr) | |
| 37 | $CF(CH_3)_2$ | H | H | H | Me | $(CH_2)_2$ | - | NMR, siehe Ende Tabelle |
| 38 | $CF(CH_3)_2$ | H | H | H | Me | $(CH_2)_2$ | 4-F | NMR, siehe Ende Tabelle |
| 39 | $CF(CH_3)_2$ | H | H | H | Me | $(CH_2)_2$ | 3,5-$Me_2$ | NMR, siehe Ende Tabelle |
| 40 | $CF(CH_3)_2$ | H | H | H | Me | $(CH_2)_2$ | 4-Me | NMR, siehe Ende Tabelle |
| 41 | $CF(CH_3)_2$ | H | H | H | Me | $(CH_2)_2$ | 2-Cl | Oel |
| 42 | $CF(CH_3)_2$ | H | H | H | Me | $(CH_2)_2$ | 4-(t-Bu) | |
| 43 | $CF(CH_3)_2$ | H | H | H | Me | $(CH_2)_2$ | 3-J | Oel |
| 44 | $CF(CH_3)_2$ | H | H | H | Me | $(CH_2)_2$ | 2,5-$Me_2$ | |
| 45 | $CF(CH_3)_2$ | H | H | H | Me | $(CH_2)_2$ | 2,6-$Cl_2$ | |
| 46 | $CF(CH_3)_2$ | H | H | H | Me | $(CH_2)_2$ | 2,4,6-$Me_3$ | Oel |
| 47 | $CF(CH_3)_2$ | H | H | H | Me | $(CH_2)_2$ | 3,5-$F_2$ | |
| 48 | $CF(CH_3)_2$ | H | H | H | Me | $(CH_2)_2$ | 3-Me | Oel |
| 49 | $CF(CH_3)_2$ | H | H | H | Me | $(CH_2)_2$ | 3-$CF_3$ | Oel |
| 50 | $CF(CH_3)_2$ | H | H | H | Me | $(CH_2)_2$ | 3-Br | |
| 51 | $CF(CH_3)_2$ | H | H | H | Me | $(CH_2)_2$ | 3,5-$Me_2$, 4-J | |
| 52 | $CF(CH_3)_2$ | H | H | H | Me | $(CH_2)_2$ | 3-OMe | |
| 53 | $CF(CH_3)_2$ | H | H | H | Me | $(CH_2)_2$ | 3,5-$(OMe)_2$ | |
| 54 | $CF(CH_3)_2$ | H | H | H | Me | $(CH_2)_2$ | 4-OMe | |

| Nr. | $R^1$ | $R^2$ | $R^3$ | $R^4$ | $R^5$ | A | $(X)_n$ | Phys. Daten |
|---|---|---|---|---|---|---|---|---|
| 55 | $CF_2CHF_2$ | H | H | H | Me | $(CH_2)_2$ | - | NMR, siehe Ende Tabelle |
| 56 | $CF_2CHF_2$ | H | H | H | Me | $(CH_2)_2$ | 3,5-$Me_2$ | |
| 57 | $CF_2CHF_2$ | H | H | H | Me | $(CH_2)_2$ | 3,4,5-$Me_3$ | |
| 58 | $CF_2CHF_2$ | H | H | H | Me | $(CH_2)_2$ | 3,5-$F_2$ | |
| 59 | $CF_2CHF_2$ | H | H | H | Me | $(CH_2)_2$ | 2,5-$Me_2$ | |
| 60 | $CF_2CHF_2$ | H | H | H | Me | $(CH_2)_2$ | 3-Me | |
| 61 | $CF_2CHF_2$ | H | H | H | Me | $(CH_2)_2$ | 3-$CF_3$ | |
| 62 | $CF_2CF_3$ | H | H | H | Me | $(CH_2)_2$ | - | |
| 63 | $CF_2CF_3$ | H | H | H | Me | $(CH_2)_2$ | 3,5-$Me_2$ | |
| 64 | $CF_2CF_3$ | H | H | H | Me | $(CH_2)_2$ | 3,5-$Me_2$, 4-J | |
| 65 | $CF_2CF_3$ | H | H | H | Me | $(CH_2)_2$ | 4-OMe | |
| 66 | $CF_2CF_3$ | H | H | H | Me | $(CH_2)_2$ | 4-OEt | |
| 67 | i-Pr | $NH_2$ | H | H | Me | $(CH_2)_2$ | 4-$NO_2$ | |
| 68 | Me | $NH_2$ | H | H | Me | $(CH_2)_2$ | 4-$CF_3$ | |
| 69 | $CF(CH_3)_2$ | $NH_2$ | H | H | Me | $(CH_2)_2$ | 3-$OCH_3$ | |
| 70 | $CF(CH_3)_2$ | CHO | H | H | Me | $(CH_2)_2$ | 3-$OC_2H_5$ | |
| 71 | $CF_2CHF_2$ | CHO | H | H | Me | $(CH_2)_2$ | 2-CN | |
| 72 | $CH_2CH_2Cl$ | CHO | H | H | Me | $(CH_2)_2$ | 4-J | |
| 73 | $CF(CH_3)_2$ | Me | H | H | Me | $(CH_2)_2$ | 3,5-$Me_2$ | |
| 74 | $C(CH_3)_2Cl$ | Me | H | H | Me | $(CH_2)_2$ | - | |
| 75 | $CF(CH_3)_2$ | Et | H | H | Me | $(CH_2)_2$ | 3,5-$F_2$ | |
| 76 | Et | Pr | H | H | Me | $(CH_2)_2$ | 2,6-$Cl_2$ | |
| 77 | i-Pr | Me | Me | H | Me | $(CH_2)_2$ | 3-F | |
| 78 | i-Pr | Et | Et | H | Me | $(CH_2)_2$ | 3,5-$Me_2$ | |
| 79 | i-Pr | H | H | Me | Me | $(CH_2)_2$ | - | |
| 80 | $CF(CH_3)_2$ | H | H | Me | Me | $(CH_2)_2$ | 3,5-$Me_2$ | |
| 81 | $CF_3$ | H | H | Me | Et | $(CH_2)_2$ | 3-Me | |
| 82 | i-Pr | H | H | Et | H | $(CH_2)_2$ | 2,5-$Me_2$ | |
| 83 | $CF(CH_3)_2$ | H | H | Et | Me | $(CH_2)_2$ | 2,4-$Cl_2$ | |
| 84 | $CF_2CHF_2$ | H | H | Br | Me | $(CH_2)_2$ | 3,5-$F_2$ | |

| Nr. | $R^1$ | $R^2$ | $R^3$ | $R^4$ | $R^5$ | A | $(X)_n$ | Phys. Daten |
|---|---|---|---|---|---|---|---|---|
| 85 | $CF_2CF_2CF_3$ | H | H | $n-C_4H_9$ | Me | $(CH_2)_2$ | $3-CF_3$ | |
| 86 | $CF(CH_3)_2$ | H | H | H | H | $(CH_2)_2$ | - | Oel |
| 87 | i-Pr | H | H | H | H | $(CH_2)_2$ | - | Oel |
| 88 | $CF(CH_3)_2$ | H | H | H | H | $(CH_2)_2$ | $3,5-Me_2$ | |
| 89 | i-Pr | H | H | H | H | $(CH_2)_2$ | $3,5-Me_2$ | |
| 90 | $CF(CH_3)_2$ | H | H | H | Et | $(CH_2)_2$ | $3,5-Me_2$ | |
| 91 | $CF(CH_3)_2$ | H | H | H | Et | $(CH_2)_2$ | - | Oel |
| 92 | $CF(CH_3)_2$ | H | H | H | n-Pr | $(CH_2)_2$ | $3,5-Me_2$ | |
| 93 | $CF(CH_3)_2$ | H | H | H | n-Pr | $(CH_2)_2$ | - | |
| 94 | i-Pr | H | H | H | H | $(CH_2)_3$ | - | |
| 95 | i-Pr | H | H | H | H | $(CH_2)_3$ | $3,5-Me_2$ | |
| 96 | Me | H | H | H | H | $(CH_2)_3$ | $3,4,5-Me_3$ | |
| 97 | Et | H | H | H | H | $(CH_2)_3$ | $3,5-Me_2$ | |
| 98 | Pr | H | H | H | H | $(CH_2)_3$ | $3,5-Me_2$, 4-J | |
| 99 | $CF(CH_3)_2$ | H | H | H | H | $(CH_2)_3$ | - | Oel |
| 100 | $CF(CH_3)_2$ | H | H | H | H | $(CH_2)_3$ | $3,5-Me_2$ | |
| 101 | $CF(CH_3)_2$ | H | H | H | H | $(CH_2)_3$ | 4-(t-Bu) | |
| 102 | $CH_3$ | H | H | H | Me | $CHMeCH_2$ | 3-OH | |
| 103 | $C_4H_9$ | H | H | H | Me | $CHMeCH_2$ | 4-OH | |
| 104 | t-Bu | H | H | H | Me | $CH_2CHMe$ | 2-OH | |
| 105 | $CF_2CF_2CF_3$ | H | H | H | Me | $CH_2CHMe$ | 3-OH | |
| 110 | i-Pr | H | H | H | Me | -CH=CH- (E)-Form | - | Oel |
| 111 | i-Pr | H | H | H | Me | -C≡C- | - | |
| 112 | i-Pr | Me | H | H | Me | $CHMeCH_2$ | $3,5-Me_2$ | Oel |
| 113 | i-Pr | Me | H | H | Et | $(CH_2)_2$ | - | Oel |
| 114 | i-Pr | Me | Me | H | Me | $CHMeCH_2$ | 3,5-Me | Oel |

| Nr. | $R^1$ | $R^2$ | $R^3$ | $R^4$ | $R^5$ | A | $(X)_n$ | Phys. Daten |
|---|---|---|---|---|---|---|---|---|
| 115 | i-Pr | Me | Me | H | Et | $(CH_2)_2$ | - | Oel |
| 116 | i-Pr | Me | Me | H | Me | $(CH_2)_2$ | - | Oel |
| 117 | i-Pr | Me | H | H | Me | $(CH_2)_2$ | - | Oel |

NMR-Daten zu einzelnen Beispielen:

Zu Beispiel 1: $^1$H-NMR (DMSO-$d_6$): δ = 1,10 (d, 3H); 1,15 (d, 6H); 1,68 (m, 1 H); 1,80 (m, 1H); 2,61 (m, 1H); 4,01 (m, 1H); 7,2 (m, 5H)

Zu Beispiel 2: $^1$H-NMR (DMSO-$d_6$): δ = 1,09 (d, 3H); 1,15 (d, 6H); 1,65 (m, 1H); 1,79 (m, 1H); 2,63 (m, 3H); 3,96 (m, 1H); 7,05 (m, 2H); 7,22 (m, 2H)

Zu Beispiel 3: $^1$H-NMR (DMSO-$d_6$): δ = 1,13 (m, 9H); 1,74 (m, 2H); 8,55 (Septett, 1 H); 2,70 (t, 2H); 4,00 (m, 1H); 7,3 (m, 4H)

Zu Beispiel 4: $^1$H-NMR ($DCCl_3$): δ = 1,22 (d, 3H); 1,26 (d, 6H); 1,79 (m, 2H); 2,29 (s, 6H); 2,60 (m, 3H); 4,01 (m, 1H); 6,78 (s, 2H); 6,82 (s, 1H)

Zu Beispiel 37: $^1$H-NMR ($CDCl_3$): δ = 1,1 (d, 3H); 1,6 (s, 3H); 1,7 (s, 3H); 2,7 (m, 3H); 7,1 (m, 3H); 7,2 (m, 3H)

Zu Beispiel 38: $^1$H-NMR ($CDCl_3$): δ = 1,1 (d, 3H); 1,6 (s, 3H); 1,7 (s, 3H); 2,6 (m, 3H); 4,0 (m, 1H); 6,9 (m, 2H); 7,1 (m, 2H)

Zu Beispiel 39: $^1$H-NMR ($CDCl_3$): δ = 1,2 (d, 3H); 1,6 (s, 3H); 1,7 (s, 3H); 1,8 (m, 2H); 2,6 (m, 2H); 4,1 (m, 1 H); 6,8 (m, 2H); 6,9 (m, 1H)

Zu Beispiel 40: $^1$H-NMR (DMSO-$d_6$): δ = 1,1 (d, 3H); 1,5 (s, 3H); 1,6 (s, 3H); 1,7 (m, 1H); 1,8 (m, 1 H); 2,2 (s, 3H), 4,0 (m, 1H); 7,1 (s, 4H)

Zu Beispiel 55: $^1$H-NMR (DMSO-$d_6$): δ = 1,1 (d, 3H); 1,7 (m, 2H); 2,6 (m, 3H); 4,0 (m, 1H); 6,7 (tt, 1H); 7,2 (m, 5H)

Tabelle 2: Verbindungen der Formel (Ia)

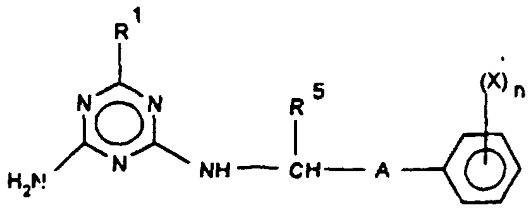

(Ia)

| Nr. | $R^1$ | $R^5$ | A | $(X)_n$ | Phys. Dat. |
|---|---|---|---|---|---|
| 2-1 | $CH_2$-c-Pr | Me | $(CH_2)_2$ | - | |
| 2-2 | $CH_2$-c-Pr | Et | $(CH_2)_2$ | - | |
| 2-3 | $CH_2$-c-Pr | Me | $(CH_2)_2$ | 3,-5-$Me_2$ | |
| 2-4 | $CH_2$-c-Pr | Et | $(CH_2)_2$ | 3,-5-$Me_2$ | |
| 2-5 | $CH(CH_3)$-c-Pr | Me | $(CH_2)_2$ | - | |
| 2-6 | 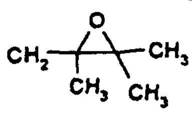 | Me | $(CH_2)_2$ | 3-Jod | |
| 2-7 | 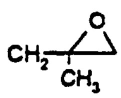 | Me | $(CH_2)_2$ | $NMe_2$ | |
| 2-8 | 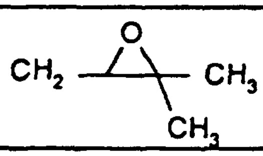 | Me | $(CH_2)_2$ | 3,5-$F_2$ | |
| 2-9 | $CH_2$-3-$CF_3$-$C_6H_3$ | Et | $(CH_2)_2$ | 3-$NMe_2$ | |
| 2-10 | $CH_2$-3-F-$C_6H_4$ | Et | $(CH_2)_2$ | 3-$NEt_2$ | |
| 2-11 | $CH_2$-2,6-$F_2$-$C_6H_3$ | Me | $(CH_2)_2$ | 3-OPh | |
| 2-12 | $CH_2$-2,6-$Cl_2$-$C_6H_3$ | Me | $(CH_2)_2$ | 3-SPh | |
| 2-13 | Ph | Et | $(CH_2)_2$ | - | |
| 2-14 | 2-$CH_3$-$C_6H_4$ | Me | $(CH_2)_2$ | - | Oel |
| 2-15 | 2-F-$C_6H_4$ | Me | $(CH_2)_2$ | - | Oel |
| 2-16 | 3-$OCH_3$-$C_6H_4$ | Et | $(CH_2)_2$ | - | |
| 2-17 | 2,4-$(CH_3)_2$-$C_6H_4$ | i-Pr | $(CH_2)_2$ | - | |
| 2-18 | 3,5-$(CH_3)_2$-$C_6H_3$ | Me | $(CH_2)_2$ | 3-Jod | |
| 2-19 | $CH_2$-$C_6H_4$ | Me | $(CH_2)_2$ | - | |

| Nr. | $R^1$ | $R^5$ | A | $(X)_n$ | Phys. Dat. |
|---|---|---|---|---|---|
| 2-20 | $CH_2$-$C_6H_4$ | Me | $(CH_2)_2$ | - | |
| 2-21 | $CH_2$-$C_6H_5$ | Et | $(CH_2)_2$ | - | |
| 2-22 | $COH(CH_3)_2$ | Me | $(CH_2)_2$ | - | Oel |
| 2-23 | $CF(CH_3)_2$ | Me | $(CH_2)_2$ | 3-Cl | Oel |
| 2-24 | $CH_2CH(CH_3)_2$ | Me | $(CH_2)_2$ | 3,5-$Me_2$ | |
| 2-25 | $CH_2CH(CH_3)_2$ | Me | $(CH_2)_2$ | - | |
| 2-26 | $CH_2CH(CH_3)_2$ | Me | $(CH_2)_2$ | 3-Me | |
| 2-27 | $CH_2CH(CH_3)_2$ | Me | $(CH_2)_2$ | 3-Jod | |
| 2-28 | $CH_2CH(CH_3)_2$ | Et | $(CH_2)_2$ | 3,5-$Me_2$ | |
| 2-29 | $CH_2CH(CH_3)_2$ | Et | $(CH_2)_2$ | - | |
| 2-30 | $CH_2CH(CH_3)_2$ | Et | $(CH_2)_2$ | 3-Me | |
| 2-31 | $CH(CH_3)C_2H_5$ | Me | $(CH_2)_2$ | - | |
| 2-32 | $CH(CH_3)C_2H_5$ | Et | $(CH_2)_2$ | - | |
| 2-33 | $CH(CH_3)C_2H_5$ | Et | $(CH_2)_2$ | 3-Me | |
| 2-34 | $CBr(CH_3)_2$ | Me | $(CH_2)_2$ | - | Oel |
| 2-35 | $C(CH_3)_2OCH_3$ | Me | $(CH_2)_2$ | - | Oel |
| 2-36 | $CCl(CH_3)_2$ | Me | $(CH_2)_2$ | | Oel |
| 2-37 | $CH(CH_3)_2$ | Me | $(CH_2)_2$ | 3-Cl | Oel |
| 2-38 | i-Pr | n-Pr | $(CH_2)_2$ | - | Oel |
| 2-39 | i-Pr | n-Pr | $(CH_2)_2$ | 3-Me | |
| 2-40 | i-Pr | i-Pr | $(CH_2)_2$ | 3,5-$Me_2$ | |
| 2-41 | $CFC(CH_3)_2$ | i-Pr | $(CH_2)_2$ | 3,5-$Me_2$ | Oel |
| 2-42 | i-Pr | $CF_3$ | $(CH_2)_2$ | $(CH_2)_2$ | |
| 2-43 | $CF(CH_3)_2$ | $CF_3$ | $(CH_2)_2$ | - | |
| 2-44 | i-Pr | $CF_3$ | $(CH_2)_2$ | 3-Jod | |
| 2-45 | $CF(CH_3)_2$ | $CF_3$ | $(CH_2)_2$ | 3-F | |
| 2-46 | i-Pr | $CF_2CF_3$ | $(CH_2)_2$ | - | |
| 2-47 | $CF(CH_3)_2$ | $CF_2CF_3$ | $(CH_2)_2$ | - | |
| 2-48 | $CH(CH_3)C_2H_5$ | $CF_2CHF_2$ | $(CH_2)_2$ | | |
| 2-49 | i-Pr | $CH_2CCl_3$ | $(CH_2)_2$ | - | |
| 2-50 | $CH_2CH(CH_3)_2$ | i-Pr | $(CH_2)_2$ | - | |

| Nr. | $R^1$ | $R^5$ | A | $(X)_n$ | Phys. Dat. |
|---|---|---|---|---|---|
| 2-51 | i-Pr | Et | $(CH_2)_2$ | - | Oel |
| 2-52 | i-Pr | Et | $(CH_2)_2$ | 4-Cl | Oel |
| 2-53 | i-Pr | Et | $(CH_2)_2$ | 3,5-$Me_2$ | Oel |
| 2-54 | i-Pr | Et | $(CH_2)_2$ | 3,4-$Cl_2$ | Oel |
| 2-55 | i-Pr | Et | $(CH_2)_2$ | 3-$CF_3$ | Oel |
| 2-56 | i-Pr | Et | $(CH_2)_2$ | 3-$CH_3$ | |
| 2-57 | $CF(CH_3)_2$ | Et | $(CH_2)_2$ | 4-Cl | Oel |
| 2-58 | $CF(CH_3)_2$ | Et | $(CH_2)_2$ | 3,4-$Cl_2$ | |
| 2-59 | $CF(CH_3)_2$ | Et | $(CH_2)_2$ | 3-$CF_3$ | |
| 2-60 | $CF(CH_3)_2$ | Et | $(CH_2)_2$ | 3-$CH_3$ | |
| 2-61 | $CHCH_3C_2H_5$ | Et | $(CH_2)_2$ | - | |
| 2-62 | $CH_2CH(CH_3)_2$ | Et | $(CH_2)_2$ | - | |
| 2-63 | $CF_2CHF_2$ | Et | $(CH_2)_2$ | - | Oel |
| 2-64 | i-Pr | i-Pr | $(CH_2)_2$ | - | |
| 2-65 | $CF(CH_3)_2$ | i-Pr | $(CH_2)_2$ | - | |
| 2-66 | i-Pr | i-Pr | $(CH_2)_2$ | 3-Me | |
| 2-67 | $CF(CH_3)_2$ | i-Pr | $(CH_2)_2$ | 3-Me | |
| 2-68 | $CH_2CH(CH_3)_2$ | i-Pr | $(CH_2)_2$ | - | |
| 2-69 | i-Pr | Me | $CH(CH_3)CH_2$ | - | Oel |
| 2-70 | $CF(CH_3)_2$ | Me | $CH(CH_3)CH_2$ | - | Oel |
| 2-70a | $CF(CH_3)_2$ | Me | $CH(CH_3)CH_2$ | 3-F | Oel |
| 2-71 | i-Pr | Me | $CH(CH_3)CH_2$ | 3-$CF_3$ | Oel |
| 2-72 | i-Pr | Me | $CH(CH_3)CH_2$ | 4-Me | Oel |
| 2-73 | $CF(CH_3)_2$ | Me | $CH(CH_3)CH_2$ | 3-$CF_3$ | Oel |
| 2-74 | $CF(CH_3)_2$ | Me | $CH(CH_3)CH_2$ | 4-Me | Oel |
| 2-75 | $CF(CH_3)_2$ | Me | $CH(CH_3)CH_2$ | 3-Me | Oel |
| 2-76 | i-Pr | Me | $CH(CH_3)CH_2$ | 3-Me | Oel |
| 2-77 | $CF(CH_3)_2$ | Me | $CH(CH_3)CH_2$ | 3,5-$Me_2$ | Oel |

| Nr. | $R^1$ | $R^5$ | A | $(X)_n$ | Phys. Dat. |
|---|---|---|---|---|---|
| 2-90 | $CF(CH_3)_2$ | Me | $(CH_2)_2$ | 3-$NH_2$ | |
| 2-91 | $CF(CH_3)_2$ | Et | $(CH_2)_2$ | 3-$NH_2$ | |
| 2-92 | $CF(CH_3)_2$ | Me | $(CH_2)_2$ | 3-$NHCOCH_3$ | |
| 2-93 | $CF(CH_3)_2$ | Et | $(CH_2)_2$ | 3-$NHCOCH_3$ | |
| 2-94 | $CF(CH_3)_2$ | Me | $(CH_2)_2$ | 3-NH-$COC_6H_5$ | |
| 2-95 | $CF(CH_3)_2$ | Et | $(CH_2)_2$ | 3-NH-$COC_6H_5$ | |
| 2-96 | $CF(CH_3)_2$ | Me | $(CH_2)_2$ | 3-NH-$CO_2CH_3$ | |
| 2-97 | $CF(CH_3)_2$ | Et | $(CH_2)_2$ | 3-NH-$CO_2CH_3$ | |
| 2-98 | $CF(CH_3)_2$ | Me | $(CH_2)_2$ | 3-NH-$CO_2C_2H_5$ | |
| 2-99 | $CF(CH_3)_2$ | Et | $(CH_2)_2$ | 3-NH-$CO_2C_2H_5$ | |
| 2-100 | $CF(CH_3)_2$ | Me | $(CH_2)_2$ | 3-NH-$COCCl_3$ | |
| 2-101 | $CF(CH_3)_2$ | Et | $(CH_2)_2$ | 3-NH-$COCCl_3$ | |
| 2-102 | $CF(CH_3)_2$ | Me | $(CH_2)_2$ | 3-NHMe | |
| 2-103 | $CF(CH_3)_2$ | Et | $(CH_2)_2$ | 3-NHMe | |
| 2-104 | $CF(CH_3)_2$ | Me | $(CH_2)_2$ | 3-$NMe_2$ | |
| 2-105 | $CF(CH_3)_2$ | Et | $(CH_2)_2$ | 3-$NMe_2$ | |
| 2-106 | i-Pr | Me | $(CH_2)_2$ | 3-$NH_2$ | |
| 2-107 | i-Pr | Et | $(CH_2)_2$ | 3-$NH_2$ | |
| 2-108 | i-Pr | Me | $(CH_2)_2$ | 3-$NHCOCH_3$ | |
| 2-109 | i-Pr | Et | $(CH_2)_2$ | 3-$NHCOCH_3$ | |
| 2-110 | i-Pr | Me | $(CH_2)_2$ | 3-$NHCO_2CH_3$ | |
| 2-111 | i-Pr | Et | $(CH_2)_2$ | 3-$NHCO_2CH_3$ | |

| Nr. | $R^1$ | $R^5$ | A | $(X)_n$ | Phys. Dat. |
|---|---|---|---|---|---|
| 2-112 | i-Pr | Me | $(CH_2)_2$ | NHMe | |
| 2-113 | i-Pr | Et | $(CH_2)_2$ | NHMe | |
| 2-114 | i-Pr | Me | $(CH_2)_2$ | NHEt | |
| 2-115 | $CF(CH_3)_2$ | Me | $(CH_2)_2$ | $OCH_2C_6H_5$ | |
| 2-116 | $CF(CH_3)_2$ | Et | $(CH_2)_2$ | $OCH_2C_6H_5$ | |
| 2-117 | $CF(CH_3)_2$ | Me | $(CH_2)_2$ | OH | |
| 2-118 | $CF(CH_3)_2$ | Et | $(CH_2)_2$ | OH | |
| 2-119 | $CF(CH_3)_2$ | Me | $(CH_2)_2$ | $OCOCH_3$ | |
| 2-120 | $CF(CH_3)_2$ | Et | $(CH_2)_2$ | $OCOCH_3$ | |
| 2-121 | $CF(CH_3)_2$ | Me | $(CH_2)_2$ | $OCO_2CH_3$ | |
| 2-122 | $CF(CH_3)_2$ | Et | $(CH_3)_2$ | $OCO_2CH_3$ | |
| 2-123 | $CF(CH_3)_2$ | Me | $(CH_2)_2$ | $OCOC_6H_5$ | |
| 2-124 | $CF(CH_3)_2$ | Et | $(CH_2)_2$ | $OCOC_6H_5$ | |
| 2-125 | $CF(CH_3)_2$ | Me | $(CH_2)_2$ | $OCONMe_2$ | |
| 2-126 | $CF(CH_3)_2$ | Et | $(CH_2)_2$ | $OCONMe_2$ | |
| 2-127 | $CF(CH_3)_2$ | Me | $(CH_2)_2$ | $OCOCl_3$ | |
| 2-128 | i-Pr | Me | $(CH_2)_2$ | $OCH_2C_6H_5$ | |
| 2-129 | i-Pr | Et | $(CH_2)_2$ | $OCH_2C_6H_5$ | |
| 2-130 | i-Pr | Me | $(CH_2)_2$ | OH | |
| 2-131 | i-Pr | Et | $(CH_2)_2$ | OH | |
| 2-132 | i-Pr | Me | $(CH_2)_2$ | $OCOCH_3$ | |
| 2-133 | i-Pr | Et | $(CH_2)_2$ | $OCOCH_3$ | |
| 2-134 | i-Pr | Me | $(CH_2)_2$ | $OCO_2CH_3$ | |
| 2-135 | i-Pr | Et | $(CH_2)_2$ | $OCO_2CH_3$ | |
| 2-136 | i-Pr | Me | $(CH_2)_2$ | $OCOC_6H_5$ | |
| 2-137 | i-Pr | Et | $(CH_2)_2$ | $OCOC_6H_5$ | |
| 2-138 | i-Pr | Me | $(CH_2)_2$ | $OCONMe_2$ | |
| 2-139 | i-Pr | Et | $(CH_2)_2$ | $OCONMe_2$ | |
| 2-140 | i-Pr | Me | $(CH_2)_2$ | $OCOCCl_3$ | |

B. Formulierungsbeispiele

a) Ein Stäubemittel wird erhalten, indem man 10 Gew.-Teile einer Verbindung der Formel (I) und 90 Gew.-Teile

Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I), **64** Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.

e) Ein in Wasser dispergierbares Granulat wird erhalten indem man

| | |
|---|---|
| 75 Gewichtsteile | einer Verbindung der Formel (I), |
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man

| | |
|---|---|
| 25 Gewichtsteile | einer Verbindung der Formel (I), |
| 5 " | 2,2'-dinaphthylmethan-6,6'-disulfonsaures Natrium |
| 2 " | oleoylmethyltaurinsaures Natrium, |
| 1 | Gewichtsteil Polyvinylalkohol, |
| 17 | Gewichtsteile Calciumcarbonat und |
| 50 " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

C. Biologische Beispiele

1. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. Auflaufschäden erfolgt nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise zeigen die Beispiele Nr. 1, 2, 3, 4, 6, 11, 37, 38, 39, 40, 41, 43, 48, 49, 55, 91, 99, 106, 2-22, 2-23, 2-34, 2-35, 2-36, 2-37, 2-38, 2-51, 2-69, 2-70 und 2-70a (s. Tabelle 1 und 2) im Test sehr gute herbizide Wirkung gegen Schadpflanzen wie Stellaria media, Lolium multiflorum, Matricaria inodora, Echinochloa crus-galli, Sinapis alba und Avena sativa, im Vorauflaufverfahren bei einer Aufwandmenge von 1,25 kg oder weniger Aktivsubstanz pro Hektar.

2. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt.

Die als Spritzpulver bzw. als Emulsionskonzentrate formulierten erfindungsgemäßen Verbindungen werden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wird die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise zeigen die Beispiele Nr. 1, 2, 3, 4, 5, 37, 38, 39, 40, 41, 43, 48, 49, 99, 106, 2-23, 2-34, 2-36, 2-38, 2-51, 2-69, 2-70 und 2-70a (s. Tabelle 1 und 2) im Test sehr gute herbizide Wirkung gegen Schadpflanzen wie Sinapis alba, Echinochloa crus-galli, Lolium multiflorum, Stellaria media, Matricaria inodora, Cyperus iria und Avena sativa im Nachauflaufverfahren bei einer Aufwandmenge von 1,25 kg und weniger Aktivsubstanz pro Hektar.

3. Wirkung auf Schadpflanzen in Reis

Verpflanzter und gesäter Reis sowie typische Reisunkräuter und -ungräser werden im Gewächshaus bis zum Dreiblattstadium (Echinochloa 1,5-Blatt) unter Paddyreis-Bedingungen (Anstauhöhe des Wassers: 2 - 3 cm) in geschlossenen Plastiktöpfen angezogen. Danach erfolgt die Behandlung mit den erfindungsgemäßen Verbindungen. Hierzu werden die formulierten Wirkstoffe in Wasser suspendiert, gelöst bzw. emulgiert und mittels Gießapplikation in das Anstauwasser der Test-pflanzen in unterschiedlichen Dosierungen ausgebracht. Nach der so durchgeführten Behandlung werden die Versuchspflanzen im Gewächshaus unter optimalen Wachstumsbedingungen aufgestellt und während der gesamten Versuchszeit so gehalten.

Etwa drei Wochen nach der Applikation erfolgt die Auswertung mittels optischer Bonitur der Pflanzenschäden im Vergleich zu unbehandelten Kontrollen. Die erfindungsgemäßen Verbindungen weisen sehr gute herbizide Wirkung gegen Schadpflanzen auf. Beispielsweise zeigen die Verbindungen der Beispiele Nr. 1, 2, 4, 6, 11, 38, 39, 43, 49, 99, 2-35, 2-36, 2-37, 2-38, 2-51 und 2-70 (siehe Tabellen 1 und 2) im Test sehr gute herbizide Wirkung gegen Schadpflanzen, die typisch für Reiskulturen sind, wie z.B. Cyperus monti, Echinochloa crus-galli und Sagittaria pygmaea.

4. Kulturpflanzenverträglichkeit

In weiteren Versuchen im Gewächshaus werden Samen einer größeren Anzahl von Kulturpflanzen und Unkräutern in sandigem Lehmboden ausgelegt und mit Erde abgedeckt. Ein Teil der Töpfe wird sofort wie unter Abschnitt 1 beschrieben behandelt, die übrigen im Gewächshaus aufgestellt, bis die Pflanzen zwei bis drei echte Blätter entwikkelt haben und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wird mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädig lassen. Einige Substanzen schonen darüber hinaus auch Gramineen-Kulturen wie z.B. Gerste, Weizen, Roggen, Sorghum-Hirsen, Mais oder Reis. Die Verbindungen der Formel (I) zeigen teilweise eine hohe Selektivität und eignen sich deshalb zur Bekämpfung von unerwünschten Pflanzenwuchs in landwirtschaftlichen Kulturen.

## Patentansprüche

1. Verbindungen der Formel (I) und deren Salze,

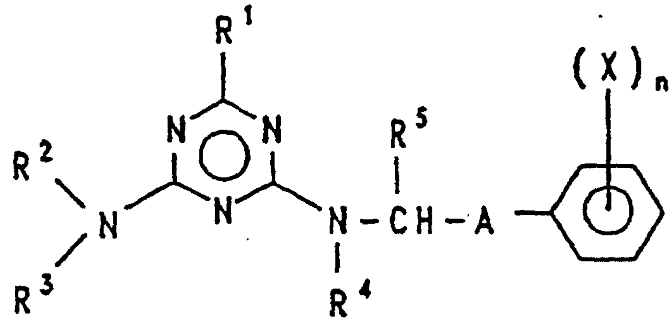

(I)

worin

$R^1$ $(C_1\text{-}C_4)$Alkyl,

das unsubstituiert oder durch einen oder mehrere Reste aus der Gruppe Halogen, $(C_1\text{-}C_4)$Alkoxy, $(C_1\text{-}C_4)$Alkylthio, $(C_1\text{-}C_4)$Alkylsulfonyl und $(C_3\text{-}C_9)$Cycloalkyl, das unsubstituiert oder substituiert ist, und Phenyl, das unsubstituiert oder durch einen oder mehrere Reste aus der Gruppe Halogen, $(C_1\text{-}C_4)$Alkyl und $(C_1\text{-}C_4)$Haloalkyl, $(C_1\text{-}C_4)$Alkoxy, $(C_1\text{-}C_4)$Haloalkoxy, $(C_1\text{-}C_4)$Alkylthio, Amino, Mono- und Di[$(C_1\text{-}C_4)$alkyl]amino, $(C_1\text{-}C_4)$Alkanoylamino, Benzoylamino, Nitro, Cyano, [$(C_1\text{-}C_4)$Alkyl]carbonyl, Formyl, Carbamoyl, Mono- und Di-[$(C_1\text{-}C_4)$alkyl]aminocarbonyl und $(C_1\text{-}C_4)$Alkylsulfonyl substituiert ist, und Heterocyclyl mit 3 bis 6 Ringatomen und 1 bis 3 Heteroringatomen aus der Gruppe N, O und S, wobei der Ring unsubstituiert oder durch einen oder mehrere Reste aus der Gruppe Halogen, $(C_1\text{-}C_4)$Alkyl und Oxo substituiert ist, substituiert ist, oder

Phenyl, das unsubstituiert oder durch einen oder mehrere Reste aus der Gruppe Halogen, $(C_1\text{-}C_4)$Alkyl, $(C_1\text{-}C_4)$Haloalkyl, $(C_1\text{-}C_4)$Alkoxy, $(C_1\text{-}C_4)$Haloalkoxy und Nitro substituiert ist,

$R^2$ und $R^3$ jeweils unabhängig voneinander Wasserstoff, Amino oder Alkylamino oder Dialkylamino mit jeweils 1 bis 4 C-Atomen im Alkylrest, einen acyclischen oder cyclischen Kohlenwasserstoffrest oder Kohlenwasserstoffoxyrest mit jeweils 1 bis 6 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder durch einen oder mehrere Reste aus der Gruppe Halogen, $(C_1\text{-}C_4)$Alkoxy, $(C_1\text{-}C_4)$Haloalkoxy, $(C_1\text{-}C_4)$Alkylthio, $(C_2\text{-}C_4)$Alkenyl, $(C_2\text{-}C_4)$Alkinyl, $(C_2\text{-}C_4)$Alkenyloxy, $(C_2\text{-}C_4)$Alkinyloxy, Hydroxy, Amino, Acylamino, Mono- und Dialkylamino, Nitrb, Carboxy, Cyano, Azido, [$(C_1\text{-}C_4)$Alkoxy]-carbonyl, [$(C_1\text{-}C_4)$Alkyl]-carbonyl, Formyl, Carbamoyl, Mono- und Di-[$(C_1\text{-}C_4)$alkyl]-aminocarbonyl, $(C_1\text{-}C_4)$Alkylsulfinyl, $(C_1\text{-}C_4)$Haloalkylsulfinyl, $(C_1\text{-}C_4)$Alkylsulfonyl, $(C_1\text{-}C_4)$Haloalkylsulfonyl und, im Falle cyclischer Reste, auch $(C_1\text{-}C_4)$Alkyl und $(C_1\text{-}C_4)$Haloalkyl substituiert ist, oder einen Acylrest oder

$R^2$ und $R^3$ gemeinsam mit dem Stickstoffatom der Gruppe $NR^2R^3$ einen heterocyclischen Rest mit 3 bis 6 Ringatomen

und 1 bis 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, $(C_1-C_4)$ Alkyl und Oxo substituiert ist,

$R^4$ Wasserstoff, Amino, Alkylamino oder Dialkylamino mit jeweils 1 bis 6 C-Atomen im Alkylrest, einen acyclischen oder cyclischen Kohlenwasserstoffrest oder Kohlenwasserstoffoxyrest mit jeweils 1 bis 6 C-Atomen oder einen Heterocyclylrest, Heterocyclyloxyrest oder Heterocyclylaminorest mit jeweils 3 bis 6 Ringatomen und 1 bis 3 Heteroringatomen aus der Gruppe N, O und S, wobei jeder der fünf letztgenannten Reste unsubstituiert oder durch einen oder mehrere Reste aus der Gruppe Halogen, $(C_1-C_4)$Alkoxy, $(C_1-C_4)$Haloalkoxy, $(C_1-C_4)$Alkylthlo, $(C_2-C_4)$Alkenyl, $(C_2-C_4)$Alkinyl, $(C_2-C_4)$Alkenyloxy, $(C_2-C_4)$Alkinyloxy, Hydroxy, Amino, Acylamino, Mono- und Dialkylamino, Nitro, Carboxy, Cyano, Azido, $[(C_1-C_4)$Alkoxy]-carbonyl, $[(C_1-C_4)$Alkyl]-carbonyl, Formyl, Carbamoyl, Mono- und Di-$[(C_1-C_4)$alkyl]-aminocarbonyl, $(C_1-C_4)$Alkylsulfinyl, $(C_1-C_4)$ Haloalkytsulfinyl, $(C_1-C_4)$Alkylsulfonyl, $(C_1-C_4)$Haloalkylsulfonyl und, im Falle cyclischer Reste, auch $(C_1-C_4)$Alkyl und $(C_1-C_4)$Haloalkyl substituiert ist,
oder einen Acylrest,

$R^5$ Wasserstoff, Halogen oder einen Rest der Formel $-B^1-Y^1$, wobei $B^1$ und $Y^1$ wie unten definiert sind,

A einen divalenten Rest der Formel $A^2$, $A^3$ oder $A^4$

$-CR^8R^9-CR^{10}R^{11}-$

$(A^2)$

$-CR^{12}=CR^{13}-$

$(A^3)$

$-CR^{14}R^{15}-CR^{16}R^{17}-CR^{18}R^{19}-$

$(A^4)$,

$R^8,R^9,R^{10},R^{11},R^{12},R^{13},R^{14},R^{15},R^{16},R^{17},R^{18},R^{19}$ jeweils unabhängig voneinander Halogen, Nitro, Cyano, Thiocyanato oder einen Rest der Formel $-B^2-Y^2$,

$(X)_n$ n Substituenten X und dabei X jeweils unabhängig voneinander Halogen, OH, $NH_2$, $NO_2$, CHO, COOH, CN, SCN oder einen Rest der Formel $-B^0-R$, wobei $B^0$ wie unten definiert ist und R $(C_1-C_6)$Alkyl, $(C_2-C_6)$Alkenyl oder $(C_2-C_6)$Alkinyl, wobei jeder der 3 letztgenannten Reste unsubstituiert oder substituiert ist, bedeutet, oder einen Rest der Formel $-B^0-R^0$, wobei $B^0$ wie unten definiert ist und $R^0$ $(C_3-C_6)$Cycloalkyl, $(C_3-C_6)$ Cycloalkenyl oder Phenyl bedeutet, wobei jeder der letztgenannten drei Reste unsubstituiert oder substituiert ist, oder
zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, $(C_1-C_4)$Alkyl und Oxo substituiert ist,

| | |
|---|---|
| n | 0, 1, 2, 3 oder 4, |
| $B^0$,$B^1$,$B^2$ | jeweils unabhängig voneinander eine direkte Bindung oder eine divalente Gruppe der Formel -O-, -S-, -CO-, -O-CO-, -CO-O-, -S-CO-, -CO-S-, -O-CO-O-, -NR'-, -NR'-CO-, -CO-NR'-, -O-CO-NR'-, -NR'-CO-O- oder -NR'-CONR''-, wobei R' und R'' unabhängig voneinander H oder ($C_1$-$C_4$)Alkyl sind, |
| $Y^1$,$Y^2$ | jeweils unabhängig voneinander H oder einen acyclischen Kohlenwasserstoffrest mit 1 bis 6 C-Atomen, einen cyclischen Kohlenwasserstoffrest mit 3 bis 6 C-Atomen oder einen heterocyclischen Rest mit 3 bis 9 Ringatomen und 1 bis 3 Heteroringatomen aus der Gruppe N, O und S, wobei jeder der drei letztgenannten Reste unsubstituiert oder durch einen oder mehrere Reste aus der Gruppe Halogen, ($C_1$-$C_4$)Alkoxy, ($C_1$-$C_4$)Haloalkoxy, ($C_1$-$C_4$)Alkylthio, ($C_2$-$C_4$)Alkenyl, ($C_2$-$C_4$) Alkinyl, ($C_2$-$C_4$)Alkenyloxy, ($C_2$-$C_4$)Alkinyloxy, Hydroxy, Amino, Acylamino, Mono- und Dialkylamino, Nitro, Carboxy, Cyano, Azido, [($C_1$-$C_4$)Alkoxy]-carbonyl, [($C_1$-$C_4$)Alkyl]-carbonyl, Formyl, Carbamoyl, Mono- und Di-[($C_1$-$C_4$)alkyl]-aminocarbonyl, ($C_1$-$C_4$)Alkylsulfinyl, ($C_1$-$C_4$)Haloalkylsulfinyl, ($C_1$-$C_4$)Alkylsulfonyl, ($C_1$-$C_4$)Haloalkylsulfonyl und, im Falle cyclischer Reste, auch ($C_1$-$C_4$)Alkyl und ($C_1$-$C_4$)Haloalkyl substituiert ist, |

bedeuten,
wobei Acyl in den Resten $R^2$, $R^3$ und $R^4$ jeweils unabhängig voneinander Formyl, ($C_1$-$C_6$)Alkylcarbonyl, Phenylcarbonyl, ($C_1$-$C_6$)Alkyloxycarbonyl, Phenyloxycarbonyl, Benzyloxycarbonyl, ($C_1$-$C_6$)Alkylsulfonyl, ($C_1$-$C_6$)Alkylsulfinyl oder N-($C_1$-$C_6$)Alkyl-1-imino-($C_1$-$C_6$)alkyl bedeutet, wobei dei Reste im Alkylteil durch ein oder mehrere Reste aus der Gruppe Halogen, ($C_1$-$C_6$)Alkoxy, Phenyl und Phenoxy und im Phenylteil durch ein oder mehrere gleiche oder verschiedene Reste aus der Gruppe Halogen, ($C_1$-$C_4$)Alkyl, ($C_1$-$C_4$)Alkoxy, ($C_1$-$C_4$)Halogenalkyl, ($C_1$-$C_4$) Halogenalkoxy und Nitro substituiert sein können.

**2.** Verbindungen nach Anspruch 1, **dadurch gekennzeichnet, dass**

| | |
|---|---|
| $R^1$ | ($C_1$-$C_4$)Alkyl, das unsubstituiert oder durch einen oder mehrere Reste aus der Gruppe Halogen, ($C_1$-$C_4$)Alkoxy, ($C_1$-$C_4$)Alkylthio, ($C_3$-$C_6$)Cycloalkyl, das unsubstituiert oder mehrere Reste aus der Gruppe Halogen, ($C_1$-$C_4$)Alkyl, ($C_1$-$C_4$)Haloalkyl, ($C_1$-$C_4$) -Alkoxy und ($C_1$-$C_4$)Haloalkoxy substituiert ist, und Phenyl, das unsubstituiert oder durch einen oder mehrere Reste aus der Gruppe Halogen, ($C_1$-$C_4$)Alkyl, ($C_1$-$C_4$)Haloalkyl, ($C_1$-$C_4$) Alkoxy, ($C_1$-$C_4$)Haloalkoxy, ($C_1$-$C_4$)Alkylthio, Nitro, Cyano, [($C_1$-$C_2$)Alkyl]carbonyl, Formyl, Carbamoyl, Mono- und Di-[($C_1$-$C_2$)alkyl]aminocarbonyl und ($C_1$-$C_4$)Alkylsulfonyl substituiert ist, substituiert ist, oder Phenyl, das unsubstituiert oder durch einen oder mehrere Reste aus der Gruppe Halogen, ($C_1$-$C_4$)Alkyl, ($C_1$-$C_4$)Haloalkyl, ($C_1$-$C_4$)-Alkoxy, ($C_1$-$C_4$)Haloalkoxy und Nitro, substituiert ist, |
| $R^2$ und $R^3$ | unabhängig voneinander Wasserstoff, Amino, Formyl, ($C_1$-$C_4$)Alkyl, Cyano-($C_1$-$C_4$)alkyl, ($C_1$-$C_4$)Alkylamino, Di-[($C_1$-$C_4$)alkyl]-amino, Halo-($C_1$-$C_4$)alkyl, Hydroxy-($C_1$-$C_4$)alkyl, ($C_1$-$C_4$)Alkoxy-($C_1$-$C_4$)alkyl, Halo($C_1$-$C_4$)alkoxy-($C_1$-$C_4$) alkyl, ($C_2$-$C_6$)Alkenyl, Halo-($C_2$-$C_6$)Alkenyl, ($C_2$-$C_6$)Alkinyl, Halo-($C_2$-$C_6$)alkinyl, ($C_1$-$C_4$)Alkylamino-($C_1$-$C_4$)alkyl, Di-[($C_1$-$C_4$)alkyl]-amino-($C_1$-$C_4$)alkyl, ($C_3$-$C_9$)Cycloalkylamino-($C_1$-$C_4$)alkyl, ($C_3$-$C_9$)Cycloalkyl, ($C_3$-$C_9$)Heterocyclyl-($C_1$-$C_4$)alkyl |

mit 3 bis 9 Ringgliedern, wobei die cyclischen Gruppen in den letztgenannten 3 Resten unsubstituiert oder durch einen oder mehrere Reste aus der Gruppe $(C_1-C_4)$Alkyl, Halogen und Cyano substituiert sind, oder Phenyl, Phenoxy, Phenylcarbonyl, Phenylcarbonyl-$(C_1-C_4)$alkyl, $(C_1-C_4)$Alkoxy-carbonyl-$(C_1-C_4)$alkyl, $(C_1-C_4)$Alkylamino-carbonyl-$(C_1-C_4)$alkyl, $(C_1-C_4)$ Alkyl-carbonyl, $(C_1-C_4)$Alkoxy-carbonyl, Aminocarbonyl, $(C_1-C_4)$Alkylamino-carbonyl, Phenoxy-$(C_1-C_4)$alkyl, Phenyl-$(C_1-C_4)$alkyl, Heterocyclyl, Heterocyclylamino, Heterocyclyloxy, Heterocyclylthio, oder einen der letztgenannten 16 Reste, der im acyclischen Teil oder im cyclischen Teil durch einen oder mehrere Reste aus der Gruppe Halogen, Nitro, Cyano, $(C_1-C_4)$Alkyl, $(C_1-C_4)$Alkoxy, $(C_1-C_4)$Alkylthio, $(C_1-C_4)$Haloalkyl, $(C_1-C_4)$Haloalkoxy, Formyl, $(C_1-C_4)$Alkyl-carbonyl, $(C_1-C_4)$Alkoxy-carbonyl, $(C_1-C_4)$Alkoxy, substituiert ist, wobei Heterocyclyl in den Resten jeweils 3 bis 9 Ringatome und 1 bis 3 Heteroringatome aus der Gruppe N, O und S enthält, oder

$R^2$ und $R^3$ gemeinsam mit dem Stickstoffatom der Gruppe $NR^2R^3$ einen heterocyclischen Rest mit 3 bis 6 Ringatomen und 1 bis 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, $(C_1-C_4)$ Alkyl und Oxo substituiert ist,

$R^4$ Wasserstoff, Amino, Formyl, $(C_1-C_4)$Alkyl, Cyano-$(C_1-C_4)$alkyl, $(C_1-C_4)$Alkylamino, Di-[$(C_1-C_4)$alkyl]-amino, Halo-$(C_1-C_4)$ alkyl, Hydroxy-$(C_1-C_4)$alkyl, $(C_1-C_4)$Alkoxy-$(C_1-C_4)$alkyl, Halo $(C_1-C_4)$alkoxy-$(C_1-C_4)$alkyl, $(C_2-C_6)$Alkenyl, Halo-$(C_2-C_6)$Alkenyl, $(C_2-C_6)$Alkinyl, Halo-$(C_2-C_6)$alkinyl, $(C_1-C_4)$Alkylamino-$(C_1-C_4)$alkyl, Di-[$(C_1-C_4)$alkyl]-amino-$(C_1-C_4)$alkyl, $(C_3-C_9)$Cycloalkylamino-$(C_1-C_4)$alkyl, $(C_3-C_9)$Cycloalkyl, $(C_3-C_9)$ Heterocyclyl-$(C_1-C_4)$alkyl mit 3 bis 9 Ringgliedern, wobei die cyclischen Gruppen in den letztgenannten 3 Resten unsubstituiert oder durch einen oder mehrere Reste aus der Gruppe $(C_1-C_4)$Alkyl, Halogen und Cyano substituiert sind, oder Phenyl, Phenoxy, Phenylcarbonyl, Phenylcarbonyl-$(C_1-C_4)$alkyl, $(C_1-C_4)$Alkoxy-carbonyl-$(C_1-C_4)$alkyl, $(C_1-C_4)$Alkylamino-carbonyl-$(C_1-C_4)$alkyl, $(C_1-C_4)$Alkyl-carbonyl, $(C_1-C_4)$Alkoxy-carbonyl, Aminocarbonyl, $(C_1-C_4)$Alkylamino-carbonyl, Phenoxy-$(C_1-C_4)$alkyl, Phenyl-$(C_1-C_4)$alkyl, Heterocyclyl, Heterocyclylamino, Heterocyclyloxy, Heterocyclylthio oder einen der letztgenannten 16 Reste, der im acyclischen Teil oder im cyclischen Teil durch einen oder mehrere Reste aus der Gruppe Halogen, Nitro, Cyano, $(C_1-C_4)$Alkyl, $(C_1-C_4)$Alkoxy, $(C_1-C_4)$ Alkylthio, $(C_1-C_4)$Haloalkyl, $(C_1-C_4)$Haloalkoxy, Formyl, $(C_1-C_4)$Alkyl-carbonyl, $(C_1-C_4)$Alkoxy-carbonyl, $(C_1-C_4)$Alkoxy, substituiert ist, wobei Heterocyclyl in den Resten jeweils 3 bis 9 Ringatome und 1 bis 3 Heteroringatome aus der Gruppe N, O und S enthält,

$R^5$ Wasserstoff, $(C_1-C_4)$Alkyl, $(C_1-C_4)$Haloalkyl oder $(C_3-C_6)$Cycloalkyl,

A einen divalenten Rest der genannten Formel $A^2$, $A^3$ oder $A^4$,

$R^8,R^9,R^{10},R^{11},R^{12},R^{13},R^{14},R^{15},R^{16},R^{17},R^{18},R^{19}$ jeweils unabhängig voneinander Wasserstoff, Halogen, $(C_1-C_8)$Alkyl, $(C_2-C_6)$Alkenyl, $(C_2-C_6)$Alkinyl, $(C_3-C_9)$Cycloalkyl, $(C_3-C_9)$Cycloalkenyl, Phenyl, Heterocyclyl mit 3 bis 6 Ringatomen und 1 bis 3 Heteroringatomen aus der Gruppe N, O und S, wobei jeder der letztgenannten 7 Reste unsubstituiert oder durch einen oder mehrere Reste aus der Gruppe Halogen, $(C_1-C_4)$Alkoxy, $(C_1-C_4)$Haloalkoxy, $(C_1-C_4)$Alkylthio, Amino, Mono- und Di[$(C_1-C_4)$alkyl]amino, $(C_1-C_4)$Alkanoylamino, Benzoylamino, Nitro, Cyano, [$(C_1-C_4)$Alkyl]carbonyl, Formyl, Carbamoyl, Mono- und Di-[$(C_1-C_4)$alkyl]aminocarbonyl, $(C_1-C_4)$Alkylsulfonyl und, im Fall cyclischer Reste, auch $(C_1-C_4)$Alkyl und $(C_1-C_4)$Haloalkyl substituiert ist,

$(X)_n$ n Substituenten X und dabei X jeweils unabhängig voneinander Halogen, Hydroxy, Amino, Nitro, Formyl, Carboxy, Cyano, Thiocyanato, $(C_1-C_4)$Alkyl, Cyano-$(C_1-C_4)$alkyl, $(C_1-C_4)$Alkoxy, $(C_1-C_4)$Alkylamino, Di-[$(C_1-C_4)$alkyl]-amino, Halo-$(C_1-C_4)$alkyl, Hydroxy-$(C_1-C_4)$alkyl, $(C_1-C_4)$Alkoxy-$(C_1-C_4)$ alkyl, Halo$(C_1-C_4)$alkoxy-$(C_1-C_4)$alkyl, $(C_1-C_4)$Alkylthio, Halo-$(C_1-C_4)$alkylthio, $(C_2-C_6)$Alkenyl, Halo-$(C_2-C_6)$Alkenyl, $(C_2-C_6)$Alkinyl, Halo-$(C_2-C_6)$alkinyl, $(C_1-C_4)$Alkylamino-$(C_1-C_4)$alkyl, Di-[$(C_1-C_4)$alkyl]-amino-$(C_1-C_4)$alkyl, $(C_3-C_9)$ Cycloalkylamino-$(C_1-C_4)$alkyl, $(C_3-C_9)$Cycloalkyl, Heterocyclyl-$(C_1-C_4)$alkyl mit 3 bis 9 Ringgliedern, wobei die cyclischen Gruppen in den letztgenannten 3 Resten unsubstituiert oder durch einen oder mehrere Reste aus der Gruppe $(C_1-C_4)$Alkyl, Halogen und Cyano substituiert sind, oder

Phenyl, Phenoxy, Phenylcarbonyl, Phenylcarbonyl-$(C_1-C_4)$alkyl, $(C_1-C_4)$Alkoxy-carbonyl-$(C_1-C_4)$alkyl, $(C_1-C_4)$Alkylamino-carbonyl-$(C_1-C_4)$alkyl, $(C_1-C_4)$Alkyl-carbonyl, $(C_1-C_4)$Alkoxy-carbonyl, Aminocarbonyl, $(C_1-C_4)$Alkylamino-carbonyl, Phenoxy-$(C_1-C_4)$alkyl, Phenyl-$(C_1-C_4)$alkyl, Heterocyclyl, Heterocyclylamino, Heterocyclyloxy, Heterocyclylthio oder einen der letztgenannten 16 Reste, der im acyclischen Teil oder im cyclischen Teil durch einen oder mehrere Reste aus der Gruppe Halogen, Nitrb, Cyano, $(C_1-C_4)$Alkyl, $(C_1-C_4)$Alkoxy, $(C_1-C_4)$ Alkylthio, $(C_1-C_4)$Haloalkyl, $(C_1-C_4)$Haloalkoxy, Formyl, $(C_1-C_4)$Alkyl-carbonyl, $(C_1-C_4)$Alkoxy-carbonyl, $(C_1-C_4)$Alkoxy, substituiert ist, wobei Heterocyclyl in den Resten jeweils 3 bis 9 Ringatome und 1 bis 3 Heteroringatome aus der Gruppe N, O und S enthält, oder

zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, $(C_1-C_4)$Alkyl und Oxo substituiert ist,

bedeuten.

**3.** Verbindungen nach Anspruch 1 oder 2, **dadurch gekennzeichnet, dass**

$R^1$ $(C_1-C_4)$Alkyl, $(C_1-C_4)$Haloalkyl, Benzyl oder [$(C_3-C_6)$Cycloalkyl]-$(C_1-C_2)$alkyl,

$R^2$ und $R^3$ unabhängig voneinander Wasserstoff, Amino, Formyl, $(C_1-C_4)$Alkyl, $(C_1-C_4)$Alkoxy-$(C_1-C_4)$alkyl, $(C_2-C_6)$Alkenyl, $(C_2-C_6)$Alkinyl, $(C_1-C_4)$Alkylamino-$(C_1-C_4)$alkyl, Di-[$(C_1-C_4)$ alkyl]-amino-$(C_1-C_4)$alkyl oder Phenyl, Phenyl-$(C_1-C_4)$alkyl oder Phenoxy-carbonyl oder einen der letztgenannten drei Reste, der im Phenylteil bis zu dreifach durch Reste aus der Gruppe Halogen, $(C_1-C_4)$Alkyl, $(C_1-C_4)$Alkoxy und $(C_1-C_4)$ Alkoxy-carbonyl substituiert ist, oder

$R^2$ und $R^3$ gemeinsam mit dem Stickstoffatom der Gruppe $NR^2R^3$ einen heterocyclischen Rest mit 3 bis 6 Ringatomen und 1 bis 2 Heteroringatomen, wobei neben dem N-Atom das

gegebenenfalls weitere Heteroringatom aus der Gruppe N und O ausgewählt ist und der Rest unsubstituiert oder durch einen oder mehrere Reste aus der Gruppe Halogen, $(C_1\text{-}C_4)$ Alkyl und Oxo substituiert ist,

$R^4$ Wasserstoff, Amino, Formyl, $(C_1\text{-}C_4)$Alkyl, Di-[$(C_1\text{-}C_4)$alkyl]-amino, $(C_1\text{-}C_4)$Alkoxy-$(C_1\text{-}C_4)$alkyl, $(C_2\text{-}C_6)$Alkenyl, $(C_2\text{-}C_6)$ Alkinyl, $(C_1\text{-}C_4)$Dialkylamino-$(C_1\text{-}C_4)$alkyl, Phenyl, Phenoxy-$(C_1\text{-}C_4)$alkyl, Phenyl-$(C_1\text{-}C_4)$alkyl, Phenoxy-carbonyl, Phenylamino-carbonyl oder einen der letztgenannten fünf Reste, der im Phenylteil einfach bis dreifach durch Reste aus der Gruppe Halogen, $(C_1\text{-}C_4)$Alkyl, $(C_1\text{-}C_4)$Alkoxy und $(C_1\text{-}C_4)$Alkoxy-carbonyl substituiert ist,

$R^5$ Wasserstoff, $(C_1\text{-}C_4)$Alkyl, $(C_1\text{-}C_4)$Halogenalkyl oder $(C_3\text{-}C_6)$ Cycloalkyl,

A einen divalenten Rest der genannten Formel $A^2$, $A^3$ oder $A^4$,

$R^8,R^9,R^{10},R^{11},R^{12},R^{13},R^{14},R^{15},R^{16},R^{17},R^{18},R^{19}$ jeweils unabhängig voneinander Wasserstoff, Halogen oder $(C_1\text{-}C_4)$Alkyl,

$(X)_n$ n Substituenten X und dabei X jeweils unabhängig voneinander Halogen, Hydroxy, $(C_1\text{-}C_4)$Alkyl oder $(C_1\text{-}C_4)$Alkoxy

bedeuten.

4. Verbindungen nach einem der Ansprüche 1 bis 3, **dadurch gekennzeichnet, dass** $R^2$ und $R^3$ unabhängig voneinander Wasserstoff bedeuten.

5. Verbindungen nach Anspruch 1 bis 3, **dadurch gekennzeichnet, dass** $R^4$ Wasserstoff bedeutet.

6. Verbindungen nach einem der Ansprüche 1 bis 3, **dadurch gekennzeichnet, dass** A einen divalenten Rest der Formel $CH_2CH_2$ bedeutet.

7. Verfahren zur Herstellung von Verbindungen der Formel (I) oder deren Salze, wie sie nach einem der Ansprüche 1 bis 6 definiert sind, **dadurch gekennzeichnet, dass** man

a) eine Verbindung der Formel (II)

$$R^1 - Fu \quad (II)$$

worin Fu eine funktionelle Gruppe aus der Gruppe Carbonsäureester, Carbonsäureorthoester, Carbonsäurechlorid, Carbonsäureamid, Carbonsäureanhydrid und Trichlormethyl bedeutet,
mit einem Biguanidid der Formel (III) oder einem Säureadditionssalz hiervon

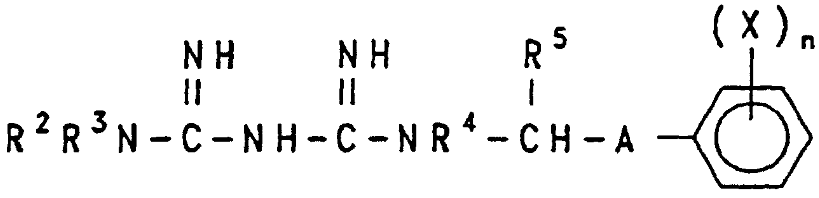

(III)

umsetzt oder

b) eine Verbindung der Formel (IV),

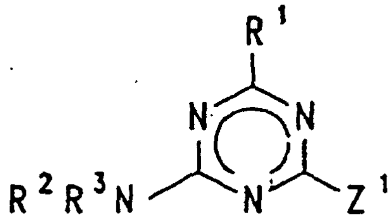

(IV)

worin $Z^1$ einen austauschfähigen Rest oder eine Abgangsgruppe bedeutet, mit einem geeigneten Amin der Formel (V) oder einem Säureadditionssalz hiervon

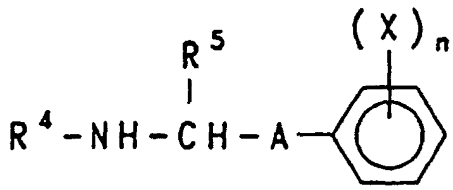

(V)

umsetzt,

wobei in den Formeln (II), (III), (IV) und (V) die Reste $R^1$, $R^2$, $R^3$, $R^4$, $R^5$, A und X sowie n wie in Formel (I) definiert sind.

8. Herbizides oder pflanzenwachstumsregulierende Mittel, **dadurch gekennzeichnet, daß** es ein oder mehrere Verbindungen der Formel (I) oder deren Salz nach einem der Ansprüche 1 bis 6 und im Pflanzenschutz übliche Formulierungshilfsmittel enthält.

9. Verfahren zur Bekämpfung von Schadpflanzen oder zur Wachstumsregulierung von Pflanzen, **dadurch gekennzeichnet, dass** man eine wirksame Menge von einer oder mehreren Verbindungen der Formel (I) oder deren Salz nach einem der Ansprüche 1 bis 6 auf die Pflanzen, Pflanzensamen oder die Anbauflache appliziert.

10. Verwendung von Verbindungen der Formel (I) oder deren Salz nach einem der Ansprüche 1 bis 6 als Herbizide oder Pflanzenwachstumsregulatoren.

**Claims**

1. Compounds of the formula (I) and their salts

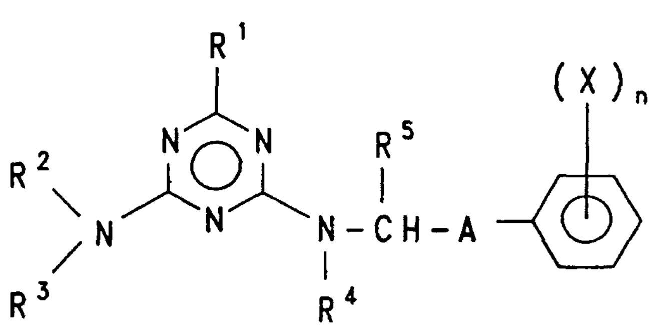

(I)

wherein

$R^1$ is $(C_1-C_4)$alkyl,

which is unsubstituted or substituted by one or more radicals from the group consisting of halogen, $(C_1-C_4)$alkoxy, $(C_1-C_4)$alkylthio, $(C_1-C_4)$alkylsulfonyl, $(C_3-C_9)$cycloalkyl which is unsubstituted or substituted, and phenyl which is unsubstituted or substituted by one or more radicals from the group consisting of halogen, $(C_1-C_4)$alkyl and $(C_1-C_4)$haloalkyl, $(C_1-C_4)$alkoxy, $(C_1-C_4)$haloalkoxy, $(C_1-C_4)$alkylthio, amino, mono- and di[$(C_1-C_4)$alkyl]amino, $(C_1-C_4)$alkanoylamino, benzoylamino, nitro, cyano, [$(C_1-C_4)$alkyl]carbonyl, formyl, carbamoyl, mono- and di-[$(C_1-C_4)$alkyl]aminocarbonyl and $(C_1-C_4)$alkylsulfonyl, and heterocyclyl having 3 to 6 ring atoms and 1 to 3 hetero ring atoms from the group consisting of N, O and S, the ring being unsubstituted or substituted by one or more radicals from the group consisting of halogen, $(C_1-C_4)$alkyl and oxo, or

phenyl which is unsubstituted or substituted by one or more radicals from the group consisting of halogen, $(C_1-C_4)$alkyl, $(C_1-C_4)$haloalkyl, $(C_1-C_4)$alkoxy, $(C_1-C_4)$haloalkoxy and nitro,

$R^2$ and $R^3$ each independently of one another are hydrogen, amino or alkylamino or dialkylamino each having 1 to 4 carbon atoms in the alkyl radical, an acyclic or cyclic hydrocarbon radical or hydrocarbon-oxy radical each having 1 to 6 carbon atoms or a heterocyclyl radical, heterocyclyloxy radical or heterocyclylamino radical each having 3 to 6 ring atoms and 1 to 3 hetero ring atoms from the group consisting of N, O and S, each of the five last mentioned radicals being unsubstituted or substituted by one or more radicals from the group consisting of halogen, $(C_1-C_4)$alkoxy, $(C_1-C_4)$haloalkoxy, $(C_1-C_4)$alkylthio, $(C_2-C_4)$alkenyl, $(C_2-C_4)$alkynyl, $(C_2-C_4)$alkenyloxy, $(C_2-C_4)$alkynyloxy, hydroxyl, amino, acylamino, mono- and dialkylamino, nitro, carboxyl, cyano, azido, [$(C_1-C_4)$alkoxy]-carbonyl, [$(C_1-C_4)$alkyl]carbonyl, formyl, carbamoyl, mono- and di-[$(C_1-C_4)$alkyl]aminocarbonyl, $(C_1-C_4)$alkylsulfinyl, $(C_1-C_4)$haloalkylsulfinyl, $(C_1-C_4)$alkylsulfonyl, $(C_1-C_4)$haloalkylsulfonyl and, in the case of cyclic radicals, also $(C_1-C_4)$alkyl and $(C_1-C_4)$haloalkyl,

or an acyl radical or

$R^2$ and $R^3$ together with the nitrogen atom of the group $NR^2R^3$ are a heterocyclic radical having 3 to 6 ring atoms and 1 to 2 hetero ring atoms, in addition to the nitrogen atom the further possible hetero ring atom being selected from the group consisting of N, O and S and the radical being unsubstituted or substituted by one or more radicals from the group consisting of halogen, $(C_1-C_4)$alkyl and oxo,

$R^4$ is hydrogen, amino, alkylamino or dialkylamino each having 1 to 6 carbon atoms in the alkyl radical,

an acyclic or cyclic hydrocarbon radical or hydrocarbon-oxy radical each having 1 to 6 carbon atoms or a heterocyclyl radical, heterocyclyloxy radical or heterocyclylamino radical each having 3 to 6 ring atoms and 1 to 3 hetero ring atoms from the group consisting of N, O and S, each of the five last mentioned radicals being unsubstituted or substituted by one or more radicals from the group consisting of halogen, $(C_1\text{-}C_4)$alkoxy, $(C_1\text{-}C_4)$haloalkoxy, $(C_1\text{-}C_4)$alkylthio, $(C_2\text{-}C_4)$alkenyl, $(C_2\text{-}C_4)$alkynyl, $(C_2\text{-}C_4)$alkenyloxy, $(C_2\text{-}C_4)$alkynyloxy, hydroxyl, amino, acylamino, mono-and dialkylamino, nitro, carboxyl, cyano, azido, $[(C_1\text{-}C_4)$alkoxy]carbonyl, $[(C_1\text{-}C_4)$alkyl]carbonyl, formyl, carbamoyl, mono- and di-$[(C_1\text{-}C_4)$alkyl]aminocarbonyl, $(C_1\text{-}C_4)$alkylsulfinyl, $(C_1\text{-}C_4)$haloalkylsulfinyl, $(C_1\text{-}C_4)$alkyl-sulfonyl, $(C_1\text{-}C_4)$haloalkylsulfonyl and, in the case of cyclic radicals, also $(C_1\text{-}C_4)$alkyl and $(C_1\text{-}C_4)$haloalkyl,
or an acyl radical,

$R^5$ is hydrogen, halogen or a radical of the formula $-B^1-Y^1$, $B^1$ and $Y^1$ being as defined below,

A is a divalent radical of the formula $A^1$, $A^2$, $A^3$ or $A^4$

$-CR^8R^9-CR^{10}R^{11}-$

$(A^2)$

$-CR^{12}=CR^{13}-$

$(A^3)$

$-CR^{14}R^{15}-CR^{16}R^{17}-CR^{18}R^{19}-$

$(A^4)$

$R^8$, $R^9$, $R^{10}$, $R^{11}$, $R^{12}$, $R^{13}$, $R^{14}$, $R^{15}$, $R^{16}$, $R^{17}$, $R^{18}$ and $R^{19}$ each independently of one another are halogen, nitro, cyano, thiocyanato or a radical of the formula $-B^2-Y^2$,

$(X)_n$ is n substituents X and in this case X in each case independently of one another is halogen, OH, $NH_2$, $NO_2$, CHO, COOH, CN, SCN or a radical of the formula $-B^0-R$, $B^0$ being as defined below and R being $(C_1\text{-}C_6)$alkyl, $(C_2\text{-}C_6)$alkenyl or $(C_2\text{-}C_6)$alkynyl, each of the 3 last mentioned radicals being unsubstituted orsubstituted, or a radical of the formula $-B^0-R^0$, $B^0$ being as defined below, and $R^0$ being $(C_3\text{-}C_6)$cycloalkyl, $(C_3\text{-}C_6)$cycloalkenyl or phenyl, each of the last mentioned three radicals being unsubstituted or substituted, preferably by one or more radicals from the group halogen, $(C_1\text{-}C_4)$alkyl, $(C_1\text{-}C_4)$halo-alkyl and $(C_1\text{-}C_4)$alkoxy, or
two adjacent radicals X together are a fused cyclic system having 4 to 6 ring atoms, which is carbocyclic or contains hetero ring atoms from the group

consisting of O, S and N and which is unsubstituted or substituted by one or more radicals from the group consisting of halogen, $(C_1-C_4)$alkyl and oxo,

n — is 0, 1, 2, 3 or 4,

$B^0$, $B^1$, $B^2$ — in each case independently of one another are a direct bond or a divalent group of the formula -O-, -S-, -CO-, -O-CO-, -CO-O-, -S-CO-, -CO-S-, -O-CO-O-, -NR'-, -NR'-CO-, -CO-NR'-, -O-CO-NR'-, -NR'-CO-O- or -NR'CONR"-, R' and R" independently of one another being H or $(C_1-C_4)$ alkyl,

$Y^1$, $Y^2$ — in each case independently of one another are H or an acyclic hydrocarbon radical having 1 to 6 carbon atoms, a cyclic hydrocarbon radical having 3 to 6 carbon atoms or a heterocyclic radical having 3 to 9 ring atoms and 1 to 3 hetero ring atoms from the group consisting of N, O and S, each of the three last mentioned radicals being unsubstituted or substituted by one or more radicals from the group consisting of halogen, $(C_1-C_4)$alkoxy, $(C_1-C_4)$haloalkoxy, $(C_1-C_4)$alkylthio, $(C_2-C_4)$alkenyl, $(C_2-C_4)$alkynyl, $(C_2-C_4)$alkenyloxy, $(C_2-C_4)$ alkynyloxy, hydroxyl, amino, acylamino, mono- and dialkylamino, nitro, carboxyl, cyano, azido, $[(C_1-C_4)$alkoxy]carbonyl, $[(C_1-C_4)$alkyl]carbonyl, formyl, carbamoyl, mono- and di-$[(C_1-C_4)$alkyl]aminocarbonyl, $(C_1-C_4)$alkylsulfinyl, $(C_1-C_4)$haloalkylsulfinyl, $(C_1-C_4)$alkylsulfonyl, $(C_1-C_4)$halo-alkylsulfonyl and, in the case of cyclic radicals, also $(C_1-C_4)$ alkyl and $(C_1-C_4)$haloalkyl,

each acyl in the $R^2$, $R^3$ and $R^4$ radicals being independently formyl, $(C_1-C_6)$alkylcarbonyl, phenylcarbonyl, $(C_1-C_6)$ alkyloxycarbonyl, phenyloxycarbonyl, benzyloxycarbonyl, $(C_1-C_6)$alkylsulfonyl, $(C_1-C_6)$alkylsulfinyl or N-$(C_1-C_6)$ alkyl-1-imino-$(C_1-C_6)$alkyl, it being possible for the radicals to be substituted by one or more radicals from the group consisting of halogen, $(C_1-C_6)$alkoxy, phenyl and phenoxy in the alkyl moiety and by one or more, identical or different radicals from the group consisting of halogen, $(C_1-C_4)$alkyl, $(C_1-C_4)$alkoxy, $(C_1-C_4)$haloalkyl, $(C_1-C_4)$ haloalkoxy and nitro in the phenyl moiety.

**2.** Compounds as claimed in claim 1, **characterized in that**,

$R^1$ — is $(C_1-C_4)$alkyl,
which is unsubstituted or substituted by one or more radicals from the group consisting of halogen, $(C_1-C_4)$ alkoxy, $(C_1-C_4)$alkylthio, $[(C_3-C_6)$cycloalkyl, which is unsubstituted or substituted by one or more radicals from the group consisting of halogen, $(C_1-C_4)$alkyl, $(C_1-C_4)$ haloalkyl, $(C_1-C_4)$alkoxy and $(C_1-C_4)$haloalkoxy, and phenyl which is unsubstituted or substituted by one or more radicals from the group consisting of halogen, $(C_1-C_4)$ alkyl, $(C_1-C_4)$haloalkyl, $(C_1-C_4)$alkoxy, $(C_1-C_4)$haloalkoxy, $(C_1-C_4)$alkylthio, nitro, cyano, $[(C_1-C_2)$alkyl]carbonyl, formyl, carbamoyl, mono- and di-$[(C_1-C_2)$alkyl]aminocarbonyl and $(C_1-C_4)$alkylsulfonyl, or
phenyl which is unsubstituted or substituted by one or more radicals from the group consisting of halogen, $(C_1-C_4)$alkyl, $(C_1-C_4)$haloalkyl, $(C_1-C_4)$alkoxy, $(C_1-C_4)$ haloalkoxy and nitro,

$R^2$ and $R^3$ — independently of one another are hydrogen, amino,

formyl, $(C_1-C_4)$alkyl, cyano-$(C_1-C_4)$alkyl, $(C_1-C_4)$alkylamino, di-[$(C_1-C_4)$alkyl]amino, halo-$(C_1-C_4)$alkyl, hydroxy-$(C_1-C_4)$alkyl, $(C_1-C_4)$alkoxy-$(C_1-C_4)$alkyl, halo$(C_1-C_4)$alkoxy-$(C_1-C_4)$alkyl, $(C_2-C_6)$alkenyl, halo-$(C_2-C_6)$alkenyl, $(C_2-C_6)$alkynyl, halo-$(C_2-C_6)$alkynyl, $(C_1-C_4)$alkylamino-$(C_1-C_4)$alkyl, di-[$(C_1-C_4)$alkyl]amino-$(C_1-C_4)$alkyl, $(C_3-C_9)$cycloalkylamino-$(C_1-C_4)$alkyl, $(C_3-C_9)$cycloalkyl, $(C_3-C_9)$heterocyclyl-$(C_1-C_4)$alkyl having 3 to 9 ring members, the cyclic groups in the last-mentioned three radicals being unsubstituted or substituted by one or more radicals from the group consisting of $(C_1-C_4)$alkyl, halogen and cyano, or

phenyl, phenoxy, phenylcarbonyl, phenylcarbonyl-$(C_1-C_4)$alkyl, $(C_1-C_4)$alkoxycarbonyl-$(C_1-C_4)$alkyl, $(C_1-C_4)$alkylaminocarbonyl-$(C_1-C_4)$alkyl, $(C_1-C_4)$alkylcarbonyl, $(C_1-C_4)$alkoxycarbonyl, aminocarbonyl, $(C_1-C_4)$alkylaminocarbonyl, phenoxy-$(C_1-C_4)$alkyl, phenyl-$(C_1-C_4)$alkyl, heterocyclyl, heterocyclylamino, heterocyclyloxy, heterocyclylthio, or one of the last-mentioned 16 radicals, which is substituted in the acyclic moiety or, preferably, in the cyclic moiety by one or more radicals from the group consisting of halogen, nitro, cyano, $(C_1-C_4)$alkyl, $(C_1-C_4)$alkoxy, $(C_1-C_4)$alkylthio, $(C_1-C_4)$haloalkyl, $(C_1-C_4)$haloalkoxy, formyl, $(C_1-C_4)$alkylcarbonyl, $(C_1-C_4)$alkoxycarbonyl, $(C_1-C_4)$alkoxy, heterocyclyl in the radicals in each case containing 3 to 9 ring atoms and 1 to 3 hetero ring atoms from the group consisting of N, O and S, or

$R^2$ and $R^3$ together with the nitrogen atom of the group $NR^2R^3$ are a heterocyclic radical having 3 to 6 ring atoms and 1 to 2 hetero ring atoms, in addition to the nitrogen atom the possible further hetero ring atom being selected from the group consisting of N, O and S and the radical being unsubstituted or substituted by one or more radicals from the group consisting of halogen, $(C_1-C_4)$alkyl and oxo,

$R^4$ is hydrogen, amino, formyl, $(C_1-C_4)$alkyl, cyano-$(C_1-C_4)$alkyl, $(C_1-C_4)$alkylamino, di-[$(C_1-C_4)$alkyl]amino, halo-$(C_1-C_4)$alkyl, hydroxy-$(C_1-C_4)$alkyl, $(C_1-C_4)$alkoxy-$(C_1-C_4)$alkyl, halo$(C_1-C_4)$-alkoxy-$(C_1-C_4)$alkyl, $(C_2-C_6)$alkenyl, halo-$(C_2-C_6)$alkenyl, $(C_2-C_6)$alkynyl, halo-$(C_2-C_6)$alkynyl, $(C_1-C_4)$alkylamino-$(C_1-C_4)$alkyl, di-[$(C_1-C_4)$alkyl]amino-$(C_1-C_4)$alkyl, $(C_3-C_9)$cycloalkylamino-$(C_1-C_4)$alkyl, $(C_3-C_9)$cycloalkyl, $(C_3-C_9)$heterocyclyl-$(C_1-C_4)$alkyl having 3 to 9 ring members, the cyclic groups in the last mentioned 3 radicals being unsubstituted or substituted by one or more radicals from the group consisting of $(C_1-C_4)$alkyl, halogen and cyano, or

phenyl, phenoxy, phenylcarbonyl, phenylcarbonyl-$(C_1-C_4)$alkyl, $(C_1-C_4)$alkoxycarbonyl-$(C_1-C_4)$alkyl, $(C_1-C_4)$alkylaminocarbonyl-$(C_1-C_4)$alkyl, $(C_1-C_4)$alkylcarbonyl, $(C_1-C_4)$alkoxycarbonyl, aminocarbonyl, $(C_1-C_4)$alkylaminocarbonyl, phenoxy-$(C_1-C_4)$alkyl, phenyl-$(C_1-C_4)$alkyl, heterocyclyl, heterocyclylamino, heterocyclyloxy, heterocyclylthio, or one of the last mentioned 16 radicals which is substituted in the acyclic moiety or in the cyclic moiety by one or more radicals from the group consisting of halogen, nitro, cyano, $(C_1-C_4)$alkyl, $(C_1-C_4)$alkoxy, $(C_1-C_4)$alkylthio, $(C_1-C_4)$haloalkyl, $(C_1-C_4)$haloalkoxy,

formyl, $(C_1-C_4)$alkylcarbonyl, $(C_1-C_4)$alkoxycarbonyl, $(C_1-C_4)$alkoxy, heterocyclyl in the radicals in each case containing 3 to 9 ring atoms and 1 to 3 hetero ring atoms from the group consisting of N, O and S,

$R^5$ is hydrogen, $(C_1-C_4)$alkyl, $(C_1-C_4)$haloalkyl or $(C_3-C_6)$cycloalkyl,

A is a divalent radical of said formula $A^2$, $A^3$ or $A^4$,

$R^8$,$R^9$,$R^{10}$,$R^{11}$,$R^{12}$,$R^{13}$,$R^{14}$,$R^{15}$,$R^{16}$$R^{17}$,$R^{18}$ and $R^{19}$ each independently of one another are hydrogen, halogen, $(C_1-C_8)$alkyl, $(C_2-C_6)$alkenyl, $(C_2-C_6)$alkynyl, $(C_3-C_9)$cycloalkyl, $(C_3-C_9)$cycloalkenyl, phenyl, heterocyclyl having 3 to 6 ring atoms and 1 to 3 hetero ring atoms from the group consisting of N, O and S, each of the last mentioned 7 radicals being unsubstituted or substituted by one or more radicals from the group consisting of halogen, $(C_1-C_4)$alkoxy, $(C_1-C_4)$haloalkoxy, $(C_1-C_4)$alkylthio, amino, mono- and di[$(C_1-C_4)$alkyl]amino, $(C_1-C_4)$alkanoylamino, benzoylamino, nitro, cyano, [$(C_1-C_4)$alkyl]carbonyl, formyl, carbamoyl, mono- and di-[$(C_1-C_4)$alkyl]aminocarbonyl, $(C_1-C_4)$alkylsulfonyl and, in the case of cyclic radicals, also$(C_1-C_4)$alkyl and $(C_1-C_4)$haloalkyl,

$(X)_n$ is n substituents X and in this case X in each case independently of one another is halogen, hydroxyl, amino, nitro, formyl, carboxyl, cyano, thiocyanato, $(C_1-C_4)$alkyl, cyano-$(C_1-C_4)$alkyl, $(C_1-C_4)$alkoxy, $(C_1-C_4)$alkylamino, di-[$(C_1-C_4)$alkyl]amino, halo-$(C_1-C_4)$alkyl, hydroxy-$(C_1-C_4)$alkyl, $(C_1-C_4)$alkoxy-$(C_1-C_4)$alkyl, halo$(C_1-C_4)$-alkoxy-$(C_1-C_4)$alkyl, $(C_1-C_4)$alkylthio, halo-$(C_1-C_4)$alkylthio, $(C_2-C_6)$alkenyl, halo-$(C_2-C_6)$alkenyl, $(C_2-C_6)$alkynyl, halo-$(C_2-C_6)$alkynyl, $(C_1-C_4)$alkylamino-$(C_1-C_4)$alkyl, di-[$(C_1-C_4)$alkyl]-amino-$(C_1-C_4)$alkyl, $(C_3-C_9)$cycloalkylamino-$(C_1-C_4)$alkyl, $(C_3-C_9)$cycloalkyl, heterocyclyl-$(C_1-C_4)$alkyl having 3 to 9 ring members, the cyclic groups in the last mentioned 3 radicals being unsubstituted or substituted by one or more radicals from the group consisting of $(C_1-C_4)$alkyl, halogen and cyano or phenyl, phenoxy, phenylcarbonyl, phenylcarbonyl-$(C_1-C_4)$alkyl, $(C_1-C_4)$alkoxycarbonyl-$(C_1-C_4)$alkyl, $(C_1-C_4)$alkylaminocarbonyl-$(C_1-C_4)$alkyl, $(C_1-C_4)$alkylcarbonyl, $(C_1-C_4)$alkoxycarbonyl, aminocarbonyl, $(C_1-C_4)$alkylaminocarbonyl, phenoxy-$(C_1-C_4)$alkyl, phenyl-$(C_1-C_4)$alkyl, heterocyclyl, heterocyclylamino, heterocyclyloxy, heterocyclylthio or one of the last mentioned 16 radicals, which is substituted in the acyclic moiety or in the cyclic moiety by one or more radicals from the group consisting of halogen, nitro, cyano, $(C_1-C_4)$alkyl, $(C_1-C_4)$alkoxy, $(C_1-C_4)$alkylthio, $(C_1-C_4)$haloalkyl, $(C_1-C_4)$haloalkoxy, formyl, $(C_1-C_4)$alkylcarbonyl, $(C_1-C_4)$alkoxycarbonyl, $(C_1-C_4)$alkoxy, heterocyclyl in the radicals in each case containing 3 to 9 ring atoms and 1 to 3 hetero ring atoms from the group consisting of N, O and S, or two adjacent radicals X together are a fused cyclic system having 4 to 6 ring atoms, which is carbocyclic or contains hetero ring atoms from the group consisting of O, S and N and which is unsubstituted or substituted by one or more radicals from the group consisting of halogen, $(C_1-C_4)$alkyl and oxo.

3. Compounds as claimed in claim 1 or 2, **characterized in that**

$R^1$ is $(C_1-C_4)$alkyl, $(C_1-C_4)$haloalkyl, benzyl or $[(C_3-C_6)$cycloalkyl$]-(C_1-C_2)$alkyl,

$R^2$ and $R^3$ independently of one another are hydrogen, amino, formyl, $(C_1-C_4)$alkyl, $(C_1-C_4)$alkoxy-$(C_1-C_4)$alkyl, $(C_2-C_6)$alkenyl, $(C_2-C_6)$alkynyl, $(C_1-C_4)$alkylamino-$(C_1-C_4)$alkyl, di-$[(C_1-C_4)$alkyl$]$-amino-$(C_1-C_4)$alkyl or phenyl, phenyl-$(C_1-C_4)$alkyl or phenoxycarbonyl or one of the last mentioned three radicals, which is substituted in the phenyl moiety up to three times by radicals from the group consisting of halogen, $(C_1-C_4)$alkyl, $(C_1-C_4)$alkoxy and $(C_1-C_4)$alkoxycarbonyl, or $R^2$ and $R^3$ together with the nitrogen atom of the group $NR^2R^3$ are a heterocyclic radical having 3 to 6 ring atoms and 1 to 2 hetero ring atoms, in addition to the nitrogen atom the possible further hetero ring atom being selected from the group consisting of N and O and the radical being unsubstituted or substituted by one or more radicals from the group consisting of halogen, $(C_1-C_4)$alkyl and oxo,

$R^4$ is hydrogen, amino, formyl, $(C_1-C_4)$alkyl, di-$[(C_1-C_4)$alkyl$]$ amino, $(C_1-C_4)$alkoxy-$(C_1-C_4)$alkyl, $(C_2-C_6)$alkenyl, $(C_2-C_6)$alkynyl, $(C_1-C_4)$dialkylamino-$(C_1-C_4)$alkyl, phenyl, phenoxy-$(C_1-C_4)$alkyl, phenyl-$(C_1-C_4)$alkyl, phenoxycarbonyl, phenylaminocarbonyl or one of the last mentioned five radicals, which is substituted in the phenyl moiety one to three times by radicals from the group consisting of halogen, $(C_1-C_4)$alkyl, $(C_1-C_4)$alkoxy and $(C_1-C_4)$alkoxycarbonyl,

$R^5$ is hydrogen, $(C_1-C_4)$alkyl, $(C_1-C_4)$haloalkyl or $(C_3-C_6)$cycloalkyl,

A is a divalent radical of said formula $A^2$, $A^3$ or $A^4$,

$R^8$,$R^9$,$R^{10}$,$R^{11}$,$R^{12}$,$R^{13}$,$R^{14}$,$R^{15}$,$R^{16}$,$R^{17}$,$R^{18}$ and $R^{19}$ each independently of one another are hydrogen, halogen or $(C_1-C_4)$alkyl,

$(X)_n$ is n substituents X and in this case X in each case independently of one another is halogen, hydroxyl, $(C_1-C_4)$alkyl or $(C_1-C_4)$alkoxy.

**4.** Compounds as claimed in any one of claims 1 to 3, **characterized in that** $R^2$ and $R^3$ are each independently of one another hydrogen.

**5.** Compounds as claimed in claim 1 to 3, **characterized in that** $R^4$ is hydrogen.

**6.** Compounds as claimed in any one of claims 1 to 3, **characterized in that** A is a divalent radical of the formula $CH_2CH_2$.

**7.** A process for the preparation of compounds of the formula (I) or salts thereof as defined according to any one of claims 1 to 6, **characterized in that** it comprises

a) reacting a compound of the formula (II)

$$R^1 - Fu \qquad (II)$$

wherein Fu is a functional group from the group consisting of carboxylic acid ester, carboxylic acid orthoester, carboxylic acid chloride, carboxamide, carboxylic anhydride and trichloromethyl,
with a biguanidide of the formula (III) or an acid addition salt thereof

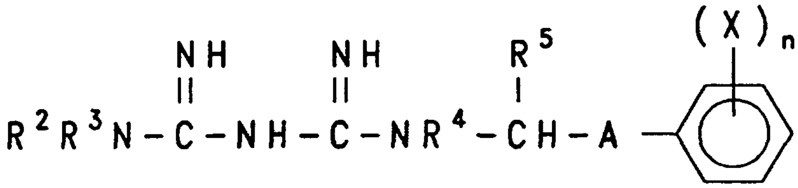

(III)

or

b) reacting a compound of the formula (IV)

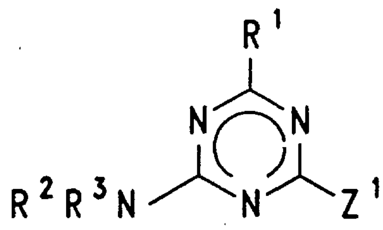

(IV)

wherein $Z^1$ is a replaceable radical or a leaving group, with a suitable amine of the formula (V) or an acid addition salt thereof

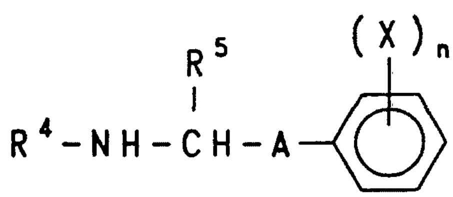

(V)

wherein the formulae (II), (III), (IV) and (V) the radicals $R^1$, $R^2$, $R^3$, $R^4$, $R^5$, A and X and also n are as defined in formula (I).

**8.** A herbicidal or plant growth-regulating composition, **characterized in that** one or more compounds of the formula (I) or their salt as claimed in any one of claims 1 to 6 and formulation aids customary in plant protection.

**9.** A process for the control of harmful plants or for regulating the growth of plants, **characterized in that** it comprises applying an effective amount of one or more compounds of the formula (I) or their salt as claimed in one of claims 1 to 6 to the plants, plant seeds or the cultivation area.

**10.** The use of compounds of the formula (I) or their salt as claimed in one of claims 1 to 6 as herbicides or plant growth regulators.

## Revendications

**1.** Composés de formule (I) et leurs sels

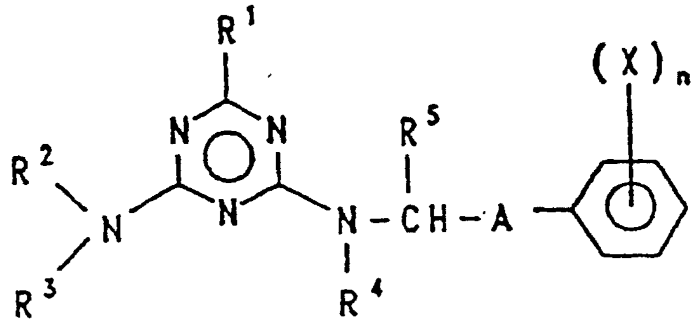

(I)

où

$R^1$ représente un groupe alkyle en $C_1$-$C_4$, qui est non substitué ou substitué par un ou plusieurs restes pris dans le groupe comprenant halogène, alkoxy en $C_1$-$C_4$, (alkyl en $C_1$-$C_4$)thio, (alkyl en $C_1$-$C_4$) sulfonyle et cycloalkyle en $C_3$-$C_9$, qui est non substitué ou substitué, et phényle, qui est non substitué ou substitué par un ou plusieurs restes pris dans le groupe comprenant halogène, alkyle en $C_1$-$C_4$ et haloalkyle en $C_1$-$C_4$, alkoxy en $C_1$-$C_4$, haloalkoxy en $C_1$-$C_4$, (alkyl en $C_1$-$C_4$)thio, amino, mono- et di(alkyl en $C_1$-$C_4$)amino, (alkanoyl en $C_1$-$C_4$)-amino, benzoylamino, nitro, cyano, (alkyl en $C_1$-$C_4$)-carbonyle, formyle, carbamoyle, mono- et di-(alkyl en $C_1$-$C_4$)aminocarbonyle et (alkyl en $C_1$-$C_4$) sulfonyle, et hétérocyclyle présentant 3 à 6 chaînons et 1 à 3 hétéroatomes pris dans le groupe comprenant un atome de N, de O et de S, le cycle est non substitué ou substitué par un ou plusieurs restes pris dans le groupe comprenant halogène, alkyle en $C_1$-$C_4$ et oxo,
ou phényle, qui est non substitué ou substitué par un ou plusieurs restes pris dans le groupe comprenant halogène, alkyle en $C_1$-$C_4$, haloalkyle en $C_1$-$C_4$, alkoxy en $C_1$-$C_4$, haloalkoxy en $C_1$-$C_4$ et nitro,

$R^2$ et $R^3$ chacun indépendamment l'un de l'autre représente un atome d'hydrogène, un groupe amino ou alkylamino ou dialkylamino présentant chacun 1 à 4 atomes de carbone dans le reste alkyle, un reste hydrocarboné ou hydrocarboxy acyclique ou cyclique présentant chacun 1 à 6 atomes de carbone ou un reste hétérocyclyle, hétérocyclyloxy ou hétérocyclylamino chacun présentant 3 à 6 chaînons et 1 à 3 hétéroatomes pris dans le groupe comprenant un atome de N, de O et de S, chacun des cinq derniers restes cités est non substitué ou substitué par un ou plusieurs restes pris dans le groupe comprenant halogène, alkoxy en $C_1$-$C_4$, haloalkoxy en $C_1$-$C_4$, (alkyl en $C_1$-$C_4$)thio, alcényle en $C_2$-$C_4$, alcynyle en $C_2$-$C_4$, alcényloxy en $C_2$-$C_4$, alcynyloxy en $C_2$-$C_4$, hydroxy, amino, acylamino, mono- et dialkylamino, nitro, carboxy, cyano, azido, (alkoxy en $C_1$-$C_4$)-carbonyle, (alkyl en $C_1$-$C_4$)-carbonyle, formyle, carbamoyle, mono- et di-(alkyl en $C_1$-$C_4$)-aminocarbonyle, (alkyl en $C_1$-$C_4$)-sulfinyle, halo(alkyl en $C_1$-$C_4$)sulfinyle, (alkyl en $C_1$-$C_4$)sulfonyle, halo(alkyl en $C_1$-$C_4$)-sulfonyle et, dans le cas de restes cycliques, aussi alkyle en $C_1$-$C_4$ et haloalkyle en $C_1$-$C_4$,
ou un reste acyle ou
$R^2$ et $R^3$ conjointement avec l'atome d'azote du groupe $NR^2R^3$ forment un reste hétérocyclique présentant 3 à 6 chaînons et 1 à 2 hétéroatomes, qui présente éventuellement, au côté de l'atome de N, d'autres hétéroatomes pris dans le groupe comprenant un atome de N, de O et de S, et le reste est non substitué ou substitué par un ou plusieurs restes pris dans le groupe comprenant halogène, alkyle en $C_1$-$C_4$ et oxo,

$R^4$ représente un atome d'hydrogène, un groupe amino, alkylamino ou dialkylamino chacun présentant 1 à 6 atomes de carbone dans le reste alkyle, un reste hydrocarboné ou hydrocarboxy acyclique ou cyclique chacun présentant 1 à 6 atomes de carbone ou un reste hétérocyclyle, hétérocyclyloxy ou hétérocyclylamino chacun présentant 3 à 6 chaînons et 1 à 3 hétéroatomes pris dans le groupe comprenant un atome de N, de O et de S, chacun des cinq derniers restes cités est non substitué ou substitué par un ou plusieurs restes pris dans le groupe comprenant halogène, alkoxy en $C_1$-$C_4$, haloalkoxy en $C_1$-$C_4$, (alkyl en $C_1$-$C_4$)thio, alcényle en $C_2$-$C_4$, alcynyle en $C_2$-$C_4$, alcényloxy, en $C_2$-$C_4$, alcynyloxy en $C_2$-$C_4$, hydroxy, amino, acylamino, mono- et dialkylamino, nitro, carboxy, cya-

no, azido, (alkoxy en $C_1$-$C_4$)-carbonyle, (alkyl en $C_1$-$C_4$)-carbonyle, formyle, carbamoyle, mono- et di- (alkyl en $C_1$-$C_4$)-aminocarbonyle, (alkyl en $C_1$-$C_4$)-sulfinyle, halo(alkyl en $C_1$-$C_4$)sulfinyle, (alkyl en $C_1$-$C_4$)-sulfonyle, halo(alkyl en $C_1$-$C_4$)sulfonyle et, dans le cas de restes cycliques, aussi alkyle en $C_1$-$C_4$ et haloalkyle en $C_1$-$C_4$, ou un reste acyle,

$R^5$ représente un atome d'hydrogène, un atome d'halogène ou un reste de formule $-B^1Y^1$, $B^1$ et $Y^1$ sont définis comme ci-dessous,

A représente un reste bivalent de formule $A^2$, $A^3$ ou $A^4$

$$-CR^8R^9-CR^{10}R^{11}-$$
**($A^2$)**

$$-CR^{12}=CR^{13}-$$
**($A^3$)**

$$-CR^{14}R^{15}-CR^{16}R^{17}-CR^{18}R^{19}-$$
**($A^4$)**

$R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$ indépendamment les uns des autres représentent un atome d'halogène, un groupe nitro, cyano, thiocyanato ou un reste de formule $-B^2-Y^2$,

$(X)_n$ représente n substituants X et chaque substituant X indépendamment l'un de l'autre représente un atome d'halogène, un groupe OH, $NH_2$, $NO_2$, CHO, COOH, CN, SCN ou un reste de formule $-B^0-R$, $B^0$ est défini comme ci-dessous et R représente un groupe alkyle en $C_1$-$C_6$, alcényle en $C_2$-$C_6$ ou alcynyle en $C_2$-$C_6$, chacun des trois derniers restes cités est non substitué ou substitué, ou représente un reste de formule $-B^0-R^0$, $B^0$ est défini comme ci-dessous et $R^0$ représente un groupe cycloalkyle en $C_3$-$C_6$, cycloalcényle en $C_3$-$C_6$ ou phényle, chacun des trois derniers restes cités est non substitué ou substitué, ou

deux restes X voisins forment conjointement un cycle condensé de 4 à 6 chaînons, qui est carbocyclique ou présente des hétéroatomes pris dans le groupe comprenant un atome de O, de S et de N, et qui est non substitué ou substitué par un ou plusieurs restes pris dans le groupe comprenant halogène, alkyle en $C_1$-$C_4$ et oxo,

n vaut 0, 1, 2, 3 ou 4,

$B^0$, $B^1$, $B^2$, chacun indépendamment l'un de l'autre représente une liaison directe ou un groupe bivalent de formule

-O-, -S-, -CO-, -O-CO-, -CO-O-, -S-CO-, -CO-S-, -O-CO-O-, -NR'-, -NR'-CO-, -CO-NR'-, -O-CO-NR'-, -NR'-CO-O- ou -NR'-CONR"-,

R' et R" indépendamment l'un de l'autre représentent un atome d'hydrogène ou un groupe alkyle en $C_1$-$C_4$,

$Y^1$, $Y^2$ chacun indépendamment l'un de l'autre représente un atome d'hydrogène ou un reste hydrocarboné acyclique présentant 1 à 6 atomes de carbone, un reste hydrocarboné cyclique présentant 3 à 6 atomes de carbone ou un reste hétérocyclique présentant 3 à 9 chaînons et 1 à 3 hétéroatomes pris dans le groupe comprenant un atome de N, de O et de S, chacun des trois derniers restes cités est non substitué ou substitué par un ou plusieurs restes pris dans le groupe comprenant halogène, alkoxy en $C_1$-$C_4$, haloalkoxy en $C_1$-$C_4$, (alkyl en $C_1$-$C_4$)thio, alcényle en $C_2$-$C_4$, alcynyle en $C_2$-$C_4$, alcényloxy en $C_2$-$C_4$, alcynyloxy en $C_2$-$C_4$, hydroxy, amino, acylamino, mono- et dialkylamino, nitro, carboxy, cyano, azido, (alkoxy en $C_1$-$C_4$)-carbonyle, (alkyl en $C_1$-$C_4$)carbonyle, formyle, carbamoyle, mono- et di-(alkyl en $C_1$-$C_4$)-aminocarbonyle, (alkyl en $C_1$-$C_4$)sulfinyle, halo(alkyl en $C_1$-$C_4$)sulfinyle, (alkyl en $C_1$-$C_4$)-sulfonyle, halo(alkyl en $C_1$-$C_4$)sulfonyle et, dans le cas de restes cycliques, est substitué aussi par alkyle en $C_1$-$C_4$ et haloalkyle en $C_1$-$C_4$,

le reste acyle dans les restes $R^2$, $R^3$ et $R^4$ à chaque fois indépendamment l'un de l'autre représente formyle, (alkyl en $C_1$-$C_6$)carbonyle, phénylcarbonyle, (alkyl en $C_1$-$C_6$)oxycarbonyle, phényloxycarbonyle, benzyloxycarbonyle, (alkyl en $C_1$-$C_6$)-sulfonyle, (alkyl en $C_1$-$C_6$)sulfinyle ou N-(alkyl en $C_1$-$C_6$)-1-imino-(alkyle en $C_1$-$C_6$), les restes dans le fragment alkyle peuvent être substitués par un ou plusieurs restes pris dans le groupe comprenant halogène, alkoxy en $C_1$-$C_6$, phényle et phénoxy, et dans le fragment phényle par un ou plusieurs restes identiques ou différents pris dans le groupe comprenant halogène, alkyle en $C_1$-$C_4$, alkoxy en $C_1$-$C_4$, halogénoalkyle en $C_1$-$C_4$, halogénoalkoxy en $C_1$-$C_4$ et nitro.

**2.** Composés selon la revendication 1, **caractérisés en ce que**

$R^1$ représente un groupe alkyle en $C_1$-$C_4$, qui est non substitué ou substitué par un ou plusieurs restes pris dans le groupe comprenant halogène, alkoxy en $C_1$-$C_4$, (alkyl en $C_1$-$C_4$)thio, cycloalkyle en $C_3$-$C_6$, qui est non substitué ou substitué par un ou plusieurs restes pris dans le groupe comprenant halogène, alkyle en $C_1$-$C_4$, haloalkyle en $C_1$-$C_4$, alkoxy en $C_1$-$C_4$ et haloalkoxy en $C_1$-$C_4$, et phényle, qui est non substitué ou substitué par un ou plusieurs restes pris dans le groupe comprenant halogène, alkyle en $C_1$-$C_4$, haloalkyle en $C_1$-$C_4$, alkoxy en $C_1$-$C_4$, haloalkoxy en $C_1$-$C_4$, (alkyl en $C_1$-$C_4$)thio, nitro, cyano, (alkyl en $C_1$-$C_2$) carbonyle, formyle, carbamoyle, mono- et di-(alkyl en $C_1$-$C_2$)-aminocarbonyle et (alkyl en $C_1$-$C_4$)-sulfonyle, ou
phényle, qui est non substitué ou substitué par un ou plusieurs restes pris dans le groupe comprenant halogène, alkyle en $C_1$-$C_4$, haloalkyle en $C_1$-$C_4$, alkoxy en $C_1$-$C_4$, haloalkoxy en $C_1$-$C_4$ et nitro,

$R^2$ et $R^3$ indépendamment l'un de l'autre représentent un atome d'hydrogène, un groupe amino, formyle, alkyle en $C_1$-$C_4$, cyanoalkyle en $C_1$-$C_4$, (alkyl en $C_1$-$C_4$)amino, di-(alkyl en $C_1$-$C_4$)-amino, haloalkyle en $C_1$-$C_4$, hydroxyalkyle en $C_1$-$C_4$, (alkoxy en $C_1$-$C_4$)-alkyle en $C_1$-$C_4$, halo(alkoxy en $C_1$-$C_4$)-alkyle en $C_1$-$C_4$, alcényle en $C_2$-$C_6$, haloalcényle en $C_2$-$C_6$, alcynyle en $C_2$-$C_6$, haloalcynyle en $C_2$-$C_6$, (alkyl en $C_1$-$C_4$) amino-alkyle en $C_1$-$C_4$, di-[(alkyl en $C_1$-$C_4$]-amino]-alkyle en $C_1$-$C_4$, cyclo(alkyl en $C_3$-$C_9$)-amino-alkyle en $C_1$-$C_4$, cycloalkyle en $C_3$-$C_9$, (hétérocyclyl en $C_3$-$C_9$)-alkyle en $C_1$-$C_4$ présentant 3 à 9 chaînons, les groupes cycliques dans les trois derniers restes cités sont non substitués ou substitués par un ou plusieurs restes pris dans le groupe comprenant alkyle en $C_1$-$C_4$, halogène et cyano, ou phényle, phénoxy, phénylcarbonyle, phénylcarbonyl-alkyle en $C_1$-$C_4$, (alkoxy en $C_1$-$C_4$)-carbonyl-alkyle en $C_1$-$C_4$, (alkyl en $C_1$-$C_4$)amino-carbonyl-alkyle en $C_1$-$C_4$, (alkyl en $C_1$-$C_4$)carbonyle, (alkoxy en $C_1$-$C_4$)-carbonyle, aminocarbonyle, (alkyl en $C_1$-$C_4$)-amino-carbonyle, phénoxylalkyle en $C_1$-$C_4$, phénylalkyle en $C_1$-$C_4$, hétérocyclyle, hétérocyclylamino, hétérocyclyloxy, hétérocyclyl-thio ou un des 16 derniers restes cités est substitué dans le fragment acyclique ou dans le fragment cyclique par un ou plusieurs restes pris dans le groupe comprenant halogène, nitro, cyano, alkyle en $C_1$-$C_4$, alkoxy en $C_1$-$C_4$, (alkyl en $C_1$-$C_4$)thio, haloalkyle en $C_1$-$C_4$, haloalkoxy en $C_1$-$C_4$, formyle, (alkyl en $C_1$-$C_4$)-carbonyle, (alkoxy en $C_1$-$C_4$)-carbonyle, alkoxy en $C_1$-$C_4$, hétérocyclyle dans les restes présente à chaque fois 3 à 9 chaînons et 1 à 3 hétéroatomes pris dans le groupe comprenant un atome de N, de O et de S, ou
$R^2$ et $R^3$ conjointement avec l'atome d'azote du groupe $NR^2R^3$ forment un reste hétérocyclique présentant 3 à 6 chaînons et 1 à 2 hétéroatomes, qui présente au côté de l'atome de N éventuellement d'autres hétéroatomes pris dans le groupe comprenant un atome de N, de O et de S et le reste est non substitué ou substitué par un ou plusieurs restes pris dans le groupe comprenant halogène, alkyle en $C_1$-$C_4$ et oxo,

$R^4$ représente un atome d'hydrogène, un groupe amino, formyle, alkyle en $C_1$-$C_4$, cyanoalkyle en $C_1$-$C_4$, (alkyl en $C_1$-$C_4$) amino, di-(alkyl en $C_1$-$C_4$)-amino, haloalkyle en $C_1$-$C_4$, hydroxyalkyle en $C_1$-$C_4$, (alkoxy en $C_1$-$C_4$)-alkyle en $C_1$-$C_4$, halo(alkoxy en $C_1$-$C_4$)-alkyle en $C_1$-$C_4$, alcényle en $C_2$-$C_6$, haloalcényle en $C_2$-$C_6$, alcynyle en $C_2$-$C_6$, haloalcynyle en $C_2$-$C_6$, (alkyl en $C_1$-$C_4$)-amino-alkyle en $C_1$-$C_4$, di-[(alkyl en $C_1$-$C_4$)-amino]-alkyle en $C_1$-$C_4$, cyclo(alkyl en $C_3$-$C_9$)-amino-alkyle en $C_1$-$C_4$, cycloalkyle en $C_3$-$C_9$, (hétérocyclyl en $C_3$-$C_9$)-alkyle en $C_1$-$C_4$ présentant 3 à 9 chaînons, les groupes cycliques des 3 derniers restes cités sont non substitués ou substitués par un ou plusieurs restes pris dans le groupe comprenant alkyle en $C_1$-$C_4$, halogène et cyano, ou phényle, phénoxy, phénylcarbonyle, phénylcarbonyl-alkyle en $C_1$-$C_4$, (alkoxy en $C_1$-$C_4$)-carbonyl-alkyle en $C_1$-$C_4$, (alkyl en $C_1$-$C_4$)amino-carbonyl-alkyle en $C_1$-$C_4$, (alkyl en $C_1$-$C_4$)-carbonyle, (alkoxy en $C_1$-$C_4$)-carbonyle, aminocarbonyle, (alkyl en $C_1$-$C_4$)-amino-carbonyle, phénoxyalkyle en $C_1$-$C_4$, phénylalkyle en $C_1$-$C_4$, hétérocyclyle, hétérocyclylamino, hétérocyclyloxy, hétérocyclylthio ou un des 16 derniers restes cités qui est substitué dans le fragment acyclique ou dans le fragment cyclique par un ou plusieurs restes pris dans le groupe comprenant halogène, nitro, cyano, alkyle en $C_1$-$C_4$, alkoxy en $C_1$-$C_4$, (alkyl en $C_1$-$C_4$)thio, haloalkyle en $C_1$-$C_4$, haloalkoxy en $C_1$-$C_4$, formyle, (alkyl en $C_1$-$C_4$)-carbonyle, (alkoxy en $C_1$-$C_4$)-carbonyle, alkoxy

en $C_1$-$C_4$, le groupe hétérocyclyle dans les restes présente à chaque fois 3 à 9 chaînons et 1 à 3 hétéroatomes pris dans le groupe comprenant un atome de N, de O et de S,

$R^5$ représente un atome d'hydrogène, un groupe alkyle en $C_1$-$C_4$, haloalkyle en $C_1$-$C_4$ ou cycloalkyle en $C_3$-$C_6$,

A représente un reste bivalent de formules $A^2$, $A^3$ ou $A^4$ citées,

$R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$ chacun indépendamment l'un de l'autre représente un atome d'hydrogène, un atome halogène, un groupe alkyle en $C_1$-$C_8$, alcényle en $C_2$-$C_6$, alcynyle en $C_2$-$C_6$, cycloalkyle en $C_3$-$C_9$, cycloalcényle en $C_3$-$C_9$, phényle, hétérocyclyle présentant 3 à 6 chaînons et 1 à 3 hétéroatomes pris dans le groupe comprenant un atome de N, de O et de S, chacun des 7 derniers restes cités est non substitué ou substitué par un ou plusieurs restes pris dans le groupe comprenant halogène, alkoxy en $C_1$-$C_4$, haloalkoxy en $C_1$-$C_4$, (alkyl en $C_1$-$C_4$)thio, amino, mono- et di(alkyl en $C_1$-$C_4$)amino, (alcanoyl en $C_1$-$C_4$)amino, benzoylamino, nitro, cyano, (alkyl en $C_1$-$C_4$)-carbonyle, formyle, carbamoyle, mono- et di-[(alkyl en $C_1$-$C_4$)]aminocarbonyle, (alkyl en $C_1$-$C_4$)-sulfonyle et, dans le cas de restes cycliques, aussi alkyle en $C_1$-$C_4$ et haloalkyle en $C_1$-$C_4$,

$(X)_n$ représente n substituants X et les substituants X chacun indépendamment l'un de l'autre représente un atome d'halogène, un groupe hydroxy, amino, nitro, formyle, carboxy, cyano, thiocyanato, alkyle en $C_1$-$C_4$, cyanoalkyle en $C_1$-$C_4$, alkoxy en $C_1$-$C_4$, (alkyl en $C_1$-$C_4$)amino, di-[(alkyl en $C_1$-$C_4$)]amino, haloalkyle en $C_1$-$C_4$, hydroxyalkyle en $C_1$-$C_4$, (alkoxy en $C_1$-$C_4$)-alkyle en $C_1$-$C_4$, halo(alkoxy en $C_1$-$C_4$)-alkyle en $C_1$-$C_4$, (alkyl en $C_1$-$C_4$)thio, halo-(alkyl en $C_1$-$C_4$)thio, alcényle en $C_2$-$C_6$, haloalcényle en $C_2$-$C_6$, alcynyle en $C_2$-$C_6$, haloalcynyle en $C_2$-$C_6$, (alkyl en $C_1$-$C_4$)amino-(alkyle en $C_1$-$C_4$), di-(alkyl en $C_1$-$C_4$)amino-(alkyle en $C_1$-$C_4$), cyclo(alkyl en $C_3$-$C_9$)amino-(alkyle en $C_1$-$C_4$), cycloalkyle en $C_3$-$C_9$, hétérocyclyl-(alkyle en $C_1$-$C_4$) présentant 3 à 9 chaînons, les groupes cycliques dans les 3 derniers restes cités sont non substitués ou substitués par un ou plusieurs restes pris dans le groupe comprenant alkyle en $C_1$-$C_4$, halogène et cyano, ou

phényle, phénoxy, phénylcarbonyle, phényl-carbonyl-alkyle en $C_1$-$C_4$, (alkoxy en $C_1$-$C_4$)-carbonyle-(alkyle en $C_1$-$C_4$), (alkyl en $C_1$-$C_4$)-amino-carbonyl-(alkyle en $C_1$-$C_4$), (alkyl en $C_1$-$C_4$)-carbonyle, (alkoxy en $C_1$-$C_4$)-carbonyle, aminocarbonyle, (alkyl en $C_1$-$C_4$)amino-carbonyle, phénoxyalkyle en $C_1$-$C_4$, phénylalkyle en $C_1$-$C_4$, hétérocyclyle, hétérocyclylamino, hétérocyclyloxy, hétérocyclylthio ou un des 16 derniers restes cités, qui est substitué dans le fragment acyclique ou dans le fragment cyclique par un ou plusieurs restes pris dans le groupe comprenant halogène, nitro, cyano, alkyle en $C_1$-$C_4$, alkoxy en $C_1$-$C_4$, (alkyl en $C_1$-$C_4$)thio, haloalkyle en $C_1$-$C_4$, haloalkoxy en $C_1$-$C_4$, formyle, (alkyl en $C_1$-$C_4$)-carbonyle, (alkoxy en $C_1$-$C_4$)-carbonyle, alkoxy en $C_1$-$C_4$, hétérocyclyle dans les restes présente à chaque fois 3 à 9 chaînons et 1 à 3 hétéroatomes pris dans le groupe comprenant un atome de N, de O et de S, ou

deux restes X voisins forment conjointement un cycle condensé présentant 4 à 6 chaînons, qui est carbocylique, ou comprend des hétéroatomes pris dans le groupe comprenant un atome de O, de S et de N, et qui est non substitué ou substitué par un ou plusieurs restes pris dans le groupe comprenant halogène, alkyle en $C_1$-$C_4$ et oxo.

3. Composés selon la revendication 1 ou 2, **caractérisés en ce que**

$R^1$ représente un groupe alkyle en $C_1$-$C_4$, haloalkyle en $C_1$-$C_4$, benzyle ou cyclo(alkyle en $C_3$-$C_6$)-alkyle en $C_1$-$C_2$,

$R^2$ et $R^3$ indépendamment l'un de l'autre représentent un atome d'hydrogène, un groupe amino, formyle, alkyle en $C_1$-$C_4$, (alkoxy en $C_1$-$C_4$)-alkyle en $C_1$-$C_4$, alcényle en $C_2$-$C_6$, alcynyle en $C_2$-$C_6$, (alkyl en $C_1$-$C_4$)amino-(alkyle en $C_1$-$C_4$), di-(alkyl en $C_1$-$C_4$)amino-(alkyle en $C_1$-$C_4$) ou phényle, phénylalkyle en $C_1$-$C_4$ ou phénoxycarbonyle ou un des trois derniers restes cités, qui est substitué jusqu'à trois fois dans le fragment phényle par des restes pris dans le groupe comprenant halogène, alkyle en, $C_1$-$C_4$, alkoxy en $C_1$-$C_4$ et (alkoxy en $C_1$-$C_4$)-carbonyle, ou

$R^2$ et $R^3$ conjointement avec l'atome d'azote du groupe $NR^2R^3$ forment un reste hétérocyclique présentant 3 à 6 chaînons et 1 à 2 hétéroatomes, éventuellement

comprenant, au côté de l'atome de N, d'autres hétéroatomes pris dans le groupe comprenant un atome de N et de O et le reste est non substitué ou substitué par un ou plusieurs restes pris dans le groupe comprenant halogène, alkyle en $C_1$-$C_4$ et oxo,

$R^4$ représente un atome d'hydrogène, un groupe amino, formyle, alkyle en $C_1$-$C_4$, di(alkyl en $C_1$-$C_4$)-amino, (alkoxy en $C_1$-$C_4$)-alkyle en $C_1$-$C_4$, alcényle en $C_2$-$C_6$, alcynyle en $C_2$-$C_6$, di(alkyl en $C_1$-$C_4$)amino-(alkyle en $C_1$-$C_4$), phényle, phénoxyalkyle en $C_1$-$C_4$, phénylalkyle en $C_1$-$C_4$, phénoxycarbonyle, phénylamino-carbonyle ou un des cinq derniers restes cités, qui est substitué dans le fragment phényle une à trois fois par des restes pris dans le groupe comprenant halogène, alkyle en $C_1$-$C_4$, alkoxy en $C_1$-$C_4$ et (alkoxy en $C_1$-$C_4$)-carbonyle,

$R^5$ représente un atome d'hydrogène, un groupe alkyle en $C_1$-$C_4$, halogénoalkyle en $C_1$-$C_4$ ou cycloalkyle en $C_3$-$C_6$,

A représente un reste bivalent de formules $A^2$, $A^3$ ou $A^4$ citées,

$R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$ chacun indépendamment l'un de l'autre représente un atome d'hydrogène, un atome halogène ou un groupe alkyle en $C_1$-$C_4$,

$(X)_n$ représente n substituants X et chaque substituant X indépendamment l'un de l'autre représente un atome d'halogène, un groupe hydroxy, alkyle en $C_1$-$C_4$ ou alkoxy en $C_1$-$C_4$.

**4.** Composés selon l'une des revendications 1 à 3, **caractérisés en ce que** $R^2$ et $R^3$ indépendamment l'un de l'autre représentent un atome d'hydrogène.

**5.** Composés selon l'une des revendications 1 à 3, **caractérisés en ce que** $R^4$ représente un atome d'hydrogène.

**6.** Composés selon l'une des revendications 1 à 3, **caractérisés en ce que** A représente un reste bivalent de formule $CH_2CH_2$.

**7.** Procédé pour la préparation de composés de formule (I) ou de leurs sels définis selon l'une des revendications 1 à 6, **caractérisé en ce qu'**on fait réagir

a) un composé de formule (II),

$$\mathbf{R^1 - Fu} \qquad \mathbf{(II)}$$

où Fu est un groupe fonctionnel pris dans le groupe comprenant des esters d'acides carboxyliques, des orthoesters d'acides carboxyliques, des chlorures d'acides carboxyliques, des amides d'acides carboxyliques, des anhydrides d'acides carboxyliques et trichlorométhyle, sur un biguanidide de formule (III) ou un de ses sels d'addition d'acide
sur un biguanidide de formule (III) ou un de ses sels d'addition d'acide

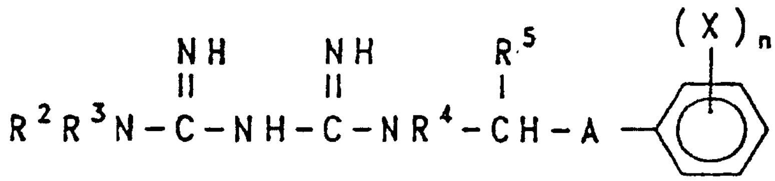

(III)

ou on fait réagir

b) un composé de formule (IV),

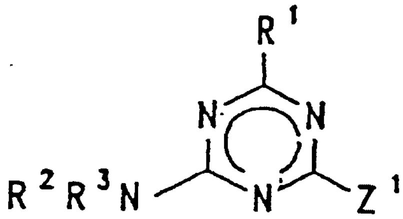

(IV)

où $Z^1$ représente un reste échangeable ou un groupe partant, sur une amine appropriée de formule (V) ou un sel d'addition d'acide de celle-ci,

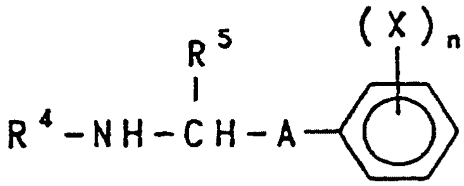

(V)

dans les formules (II), (III), (IV) et (V), les restes $R^1$, $R^2$, $R^3$, $R^4$, $A^1$, $A^2$ et X ainsi que n sont définis comme à la formule (I).

**8.** Agent herbicide ou régulateur de croissance de plantes, **caractérisé en ce qu'**il renferme un ou plusieurs composés de formule (I) ou leurs sels selon l'une des revendications 1 à 6 et des formulants usuels dans le domaine phytoprotecteur.

**9.** Procédé pour la lutte contre les mauvaises herbes ou pour la régulation de la croissance de plantes, **caractérisé en ce qu'**on applique une quantité efficace d'un ou plusieurs composés de formule (I) ou de leurs sels selon l'une des revendications 1 à 6 aux plantes, à la semence de plantes ou à la surface cultivable.

**10.** Utilisation de composés de formule (I) ou de leurs sels selon l'une des revendications 1 à 6 en tant qu'herbicides ou régulateurs de croissance végétale.